(19) Europäisches Patentamt
European Patent Office
Office européen des brevets

(11) EP 0 606 183 B1

(12) **FASCICULE DE BREVET EUROPEEN**

(45) Date de publication et mention de la délivrance du brevet:
**14.04.1999 Bulletin 1999/15**

(51) Int. Cl.$^6$: **C07C 45/51**, C07C 49/683, C07C 49/747

(21) Numéro de dépôt: **94400020.7**

(22) Date de dépôt: **05.01.1994**

(54) **Procédé de préparation d'une p-fuchsone**

Verfahren zur Herstellung eines p-Fuchsons

Process for the preparation of a p-fuchsone

(84) Etats contractants désignés:
**DE FR GB IT NL SE**

(30) Priorité: **08.01.1993 FR 9300119**
**08.01.1993 FR 9300120**
**08.01.1993 FR 9300121**
**22.09.1993 FR 9311262**

(43) Date de publication de la demande:
**13.07.1994 Bulletin 1994/28**

(60) Demande divisionnaire:
**98110051.4 / 0 890 564**

(73) Titulaire: **RHODIA CHIMIE**
**92408 Courbevoie Cédex (FR)**

(72) Inventeurs:
- **Costantini, Michel M.**
  **F-69003 Lyon (FR)**
- **Manaut, Daniel M.**
  **F-69330 Meyzieu (FR)**
- **Michelet, Daniel M.**
  **F-78860 Saint-Nom-La-Breteche (FR)**

(74) Mandataire:
**Dutruc-Rosset, Marie-Claude et al**
**RHODIA SERVICES**
**Direction de la Propriété Industrielle**
**25, quai Paul Doumer**
**92408 Courbevoie Cédex (FR)**

(56) Documents cités:
**FR-A- 2 071 464** **FR-A- 2 336 364**
**US-A- 3 649 653** **US-A- 4 078 006**

- **CHEMICAL ABSTRACTS, vol. 108, no. 17, 25 Avril 1988, Columbus, Ohio, US; abstract no. 150241s, KOZLIKOVSKII, YA.B. ET AL. 'Reaction of phenol with benzophenone in the presence of aluminium phenolate.' page 723 ;colonne 1 ; & IZV. VYSSH. UCHEBN. ZAVED., KHIM. KHIM. TEKHNOL. vol. 30, no. 7 , 1987 pages 31 - 34**
- **METHODEN DER ORGANISCHEN CHEMIE (HOUBEN - WEYL) vol. VII, no. 3B , 1979 , STUTTGART pages 458 - 462 P. GRÜNANGER 'Chinonmethide'**
- **CHEMICAL ABSTRACTS, vol. 098, no. 21, 23 Mai 1983, Columbus, Ohio, US; abstract no. 178889, PISOVA M ET AL 'Quinone methides and fuchsones. XXVII. Oxidative rearrangement of o-fuchsone to 2,2-diphenyl-1,3-benzodioxole' & COLLECT. CZECH. CHEM. COMMUN. (CCCCAK,0366547X);82; VOL.47 (12); PP.3318-27 CZECH. ACAD. SCI.;INST. ORG. CHEM. BIOCHEM.; PRAGUE; 166 10/6; CZECH. (CS)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 606 183 B1

## Description

La présente invention a pour objet un procédé de préparation d'une p-fuchsone.

Il existe dans la littérature, peu de références sur la synthèse des fuchsones.

Généralement, leur préparation est basée sur la déhydratation d'un carbinol.

Ainsi, I. S. IOFFE et al [J. Gen. Chem. USSR, 19, p.917-28 (1949)], ont décrit la préparation d'hydroxy-4 triphénylcarbinol, par réaction de $Ph_2CCl_2$ avec le phénol fondu.

H. BURTON et al [J. Chem. Soc., 3089-3090 (1955)] préconisent la synthèse de l'hydroxy-4 triphényl carbinol par addition de $Ph_2CCl_2$ dans une suspension de chlorure d'aluminium dans du sulfure de carbone et ensuite, introduction dans ce milieu réactionnel de phénol en solution dans le sulfure de carbone.

L'hydroxy-4 triphényl carbinol ainsi obtenu est ensuite déshydraté. Une technique de déshydratation décrite par I. S. IOFFE et al (loc. cit.) est de le soumettre, après addition d'acide acétique, à un traitement thermique, conduisant ainsi à la fuchsone suivante :

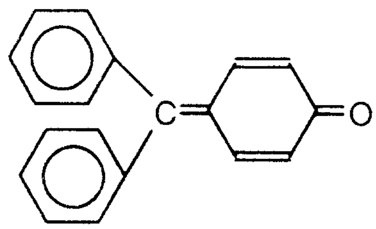

L'inconvénient dont souffrent les procédés décrits dans l'état de la technique est qu'il est quasiment impossible de mettre en oeuvre de tels procédés à l'échelle industrielle, en raison d'une matière première difficilement accessible ($Ph_2CCl_2$) et de la difficulté de manipulation de réactifs tels que le chlorure d'aluminium et le sulfure de carbone.

Par ailleurs, Kozlikovski et al [Chemical Abstracts 108, 150241s (1988)] ont décrit la condensation du phénol, de la benzophénone et du phénolate d'aluminium à 190°C. De nombreux produits sont obtenus : p-fuchsone (21,3 %), 2,4'-(diphénylméthylène)diphénol (11,1 %) et 4,4'-(diphénylméthylène)diphénol (12,1 %).

Selon US-A 3 649 653, on a obtenu un triphénylcarbinol, par irradiation d'une solution de 2,6-dialkylphénol et d'une benzophénone : le carbinol étant transformé en fuchsone par acidification.

L'objectif de la présente invention est de fournir un procédé industriel de fabrication de fuchsones et ceci avec de bons rendements réactionnels.

La présente invention a pour objet un procédé de préparation d'une p-fuchsone caractérisé par le fait qu'il consiste à faire réagir un composé phénolique présentant un atome d'hydrogène en position para du groupe hydroxyle et un composé cétonique non énolisable, en présence d'une quantité efficace d'un acide protonique présentant une fonction d'acidité - Ho d'au moins 5 et éventuellement en présence d'une quantité efficace d'un composé soufré ionisable.

Dans l'exposé qui suit de la présente invention, on entend par "p-fuchsone", tout composé chimique comportant le motif méthylène-1,4 quinone suivant :

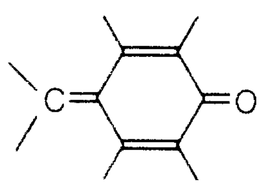

Le substrat de départ est un compose aromatique porteur d'au moins un groupe hydroxyle et présentant un atome d'hydrogène en position para du groupe hydroxyle. Par "composé aromatique", on entend la notion classique d'aromaticité telle que définie dans la littérature, notamment par Jerry MARCH, Advanced Organic Chemistry, 3ème édition, John Wiley and Sons, 1985, p.37 et suivantes.

Dans le présent texte, on désigne par "composé cétonique non énolisable", une cétone qui possède deux atomes de carbone tertiaire en position $\alpha$ du groupe carbonyle,

On appelle "composé soufré ionisable", tout composé soufré qui s'ionise en présence d'eau pour donner un

ion présentant une valence libre sur l'atome de soufre.

Une variante de l'invention, consiste à conduire le procédé de l'invention, en présence d'un additif, à savoir un composé soufré ionisable. En effet, il a été trouvé que la mise en oeuvre d'un tel composé permettait d'accroître la cinétique réactionnelle. Ainsi, on a constaté que la vitesse de formation de la fuchsone était considérablement augmentée et qu'elle pouvait être multipliée par dix ou voire même plus.

Conformément au procédé de l'invention, on prépare une p-fuchsone en faisant réagir les composés phénolique et cétonique tels que définis, en présence d'un acide et éventuellement d'un composé soufré ionisable.

La présente invention s'applique tout particulièrement aux composés phénoliques de formule générale (I) :

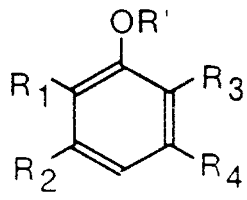
(I)

dans ladite formule (I) :

- la position en para est libre,
- $R_1$, $R_2$, $R_3$ et $R_4$, identiques ou différents représentent un atome d'hydrogène ou un substituant quelconque,
- deux groupes $R_1$ et $R_2$ et/ou $R_3$ et $R_4$ placés sur deux atomes de carbone vicinaux peuvent former ensemble et avec les atomes de carbone qui les portent un cycle,
- R' représente un atome d'hydrogène ou un radical hydrocarboné ayant de 1 à 24 atomes de carbone, qui peut être un radical aliphatique acyclique saturé ou insaturé, linéaire ou ramifié ; un radical cycloaliphatique saturé ou insaturé, monocyclique ou polycyclique ; un radical aliphatique saturé ou insaturé, linéaire ou ramifié, porteur d'un substituant cyclique.

Par substituant cyclique, on entend un carbocycle saturé, insaturé ou aromatique ayant, généralement, de 4 à 7 atomes de carbone, et de préférence 6 atomes de carbone.

Le procédé de l'invention s'applique à tout composé pnénolique répondant à la formule générale (I) et, plus particulièrement, aux composés phénoliques de formule (I) dans laquelle R' représente :

. un atome d'hydrogène
. un radical alkyle linéaire ou ramifié ayant de 1 à 6 atomes de carbone, de préférence de 1 à 4 atomes de carbone,et plus particulièrement un radical méthyle ou éthyle,
. un radical cyclohexyle,
. un radical benzyle.

Le composé phénolique de formule (I) peut être porteur d'un ou plusieurs substituants $R_1$, $R_2$, $R_3$ et $R_4$. Des exemples de substituants sont donnés ci-après mais cette liste ne présente aucun caractère limitatif. N'importe quel substituant peut être présent sur le cycle dans la mesure où il n'interfère pas au niveau du produit désiré.

Le procédé de l'invention s'applique plus preférentiellement aux composés phénoliques de formule (I) dans lesquels :

- $R_1$, $R_2$, $R_3$ et $R_4$, identiques ou différents représentent $R_0$, l'un des groupes suivants :
  . un atome d'hydrogène,
  . un radical alkyle, linéaire ou ramifié, ayant de 1 à 6 atomes de carbone, de préférence de 1 à 4 atomes de carbone, tel que méthyle, éthyle, propyle, isopropyle, butyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un groupe acyle ayant de 2 à 6 atomes de carbone,
  . un radical de formule :

$-R_5-OH$

$-R_5-COOR_6$

$-R_5-X$

$-R_5-CF_3$

dans lesdites formules, $R_5$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_6$ représente un atome d'hydrogène ou un radical alkyle linéaire ou ramifié ayant de 1 à 6 atomes de carbone ; X symbolise un atome d'halogène, de préférence un atome de chlore, de brome ou de fluor.

- $R_1$, $R_2$, $R_3$ et $R_4$, identiques ou différents représentent $R_7$, l'un des radicaux plus complexes suivants :

  . un radical carbocyclique saturé ou insaturé ayant de 4 à 7 atomes de carbone, de préférence un radical cyclohexyle,
  . un radical de formule

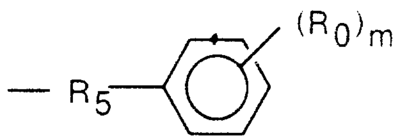

dans lequel $R_5$ représente un lien valentiel ou un radical hydrocarboné divalent, linéaire ou ramifié, saturé ou insaturé ayant de 1 à 6 atomes de carbone tel que par exemple, méthylène, éthylène, propylène, isopropylène, isopropylidène et $R_0$ ayant la signification donnée précédemment et m est un nombre entier de 0 à 4,

  . un radical $-R_5-A-R_8$ dans lequel $R_5$ a la signification donnée précédemment, $R_8$ représente un radical alkyle linéaire ou ramifié ayant de 1 à 6 atomes de carbone ou un radical de formule

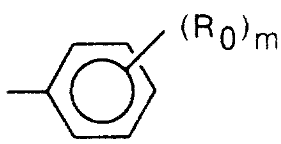

et A symbolise l'un des groupes suivants :

$-O-$, $-COO-$, $-OCOO-$, $-SO_2-$, $-CO-N(R_9)-$,

dans ces formules, $R_9$ représente un atome d'hydrogène ou un radical alkyle ayant de 1 à 4 atomes de carbone, un radical cyclohexyle ou phényle.

- deux groupes $R_1$ et $R_2$ et/ou $R_3$ et $R_4$ placés sur deux atomes de carbone vicinaux peuvent former ensemble et avec les atomes de carbone qui les portent un carbocycle insaturé ou aromatique ayant de 4 à 7 atomes de carbone et, de préférence, 6 atomes de carbone,

Parmi les composés de formule (I), on met en oeuvre plus particulièrement ceux répondant à la formule (I) dans laquelle :

- R' représente un atome d'hydrogène
- $R_1$, $R_2$, $R_3$ et $R_4$, identiques ou différents représentent l'un des groupes suivants :

. un atome d'hydrogène,
. un radical alkyle, linéaire ou ramifié ayant de 1 à 4 atomes de carbone,.
. un radical alkoxy linéaire ou ramifié ayant de 1 à 4 atomes de carbone,
. un groupe hydroxyle,
. un atome d'halogène,
. un groupe $-CF_3$
. un radical cyclohexyle,
. un radical phényle,

- deux groupes $R_1$ et $R_2$ et/ou $R_3$ et $R_4$ placés sur deux atomes de carbone vicinaux peuvent former ensemble et avec les atomes de carbone qui les portent un cycle benzénique.

Encore plus préférentiellement, on choisit les composés de formule (I) dans laquelle R' représente un atome d'hydrogène et l'un des radicaux $R_1$, $R_2$, $R_3$ et $R_4$ représente un groupe hydroxyle, un radical méthyle ou un radical méthoxy et les 3 autres représentent un atome d'hydrogène.
A titre illustratif de composés phénoliques de formule (I) susceptibles d'être mis en oeuvre dans le procédé de l'invention, on peut mentionner plus particulièrement :

- ceux répondant à la formule (I) dans laquelle $R_1$, $R_2$, $R_3$ et $R_4$ représentent un atome d'hydrogène, tels que le phénol ou l'anisole,
- ceux répondant à la formule (I) avec un substituant sur le cycle benzénique, tels que l'orthocrésol, le métacrésol, le 2-méthoxyphénol, le 2-éthylphénol, le 3-éthylphénol, 2-propylphénol, le 2-sec-butylphénol, le 2-tert-butylphénol, le 3-tert-butylphénol, le 2-méthoxyphénol, le 3-méthoxyphénol, le salicylate de méthyle, le 2-chlorophénol, le 3-chlorophénol,
- ceux répondant à la formule (I) avec deux substituants sur le cycle benzénique, tels que le 2,3-diméthylphénol, le 2,5-diméthylphénol, le 2,6-diméthylphénol, le 3,5-diméthyphénol, le 2,3-dichlorophénol, le 2,5-dichlorophénol, le 2,6-dichlorophénol, le 3,5-dichlorophénol, le 2,6-ditert-butylphénol, le 3,5-ditert-butylphénol,
- ceux répondant à la formule (I) avec trois substituants sur le cycle benzénique, tels que le 2,3,5-triméthylphénol, le 2,3,6-triméthylphénol, le 2,3,5-trichlorophenol, le 2,3,6-trichlorophénol,
- ceux répondant à la formule (I) dans laquelle $R_1$ et $R_2$ forment un cycle benzénique, tels que le 1-hydroxynaphtalène,
- ceux répondant à la formule (I) dans laquelle $R_1$ représente un radical de type $R_7$, tels que le 2-phénoxyphénol, le 3-phénoxyphénol.

Parmi les composés phénoliques de formule (I) qui pourront être mis en oeuvre dans le procédé de l'invention, on peut citer à titre non limitatif, le phénol, l'orthocrésol, le métacrésol.
Comme mentionné précédemment, la caractéristique du composé cétonique intervenant dans le procédé de l'invention est qu'il s'agit d'une cétone non énolisable.
Plus spécifiquement, il répond à la formule (II) suivante :

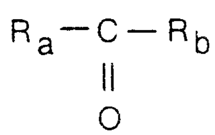
(II)

dans ladite formule (II) :

- $R_a$ et $R_b$, identiques ou différents sont des radicaux hydrocarbonés ayant chacun de 3 à 30 atomes de carbone ; les atomes de carbone de chaque radical $R_a$ et $R_b$ en position $\alpha$ par rapport au groupe carbonyle étant des carbones tertiaires.

A titre d'exemples de radicaux $R_a$ et $R_b$ convenant bien à la présente invention, on peut citer entre autres, les radicaux alkyle ramifiés ayant au moins 3 atomes de carbone et les radicaux aryle ayant au moins 6 atomes de carbone et plus particulièrement les radicaux tert-butyle, tert-pentyle, tert-hexyle ou phényle éventuellement substitué.
Les composés cétoniques convenant à la présente invention peuvent être représenter plus précisément par la formule générale (IIa) suivante :

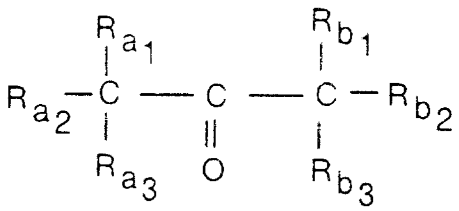

(IIa)

dans ladite formule (IIa) :

- $R_{a1}$, $R_{a2}$, $R_{a3}$ et $R_{b1}$, $R_{b2}$, $R_{b3}$ identiques ou différents, représentent des radicaux alkyle linéaires ou ramifiés ayant de 1 à 10 atomes de carbone, des radicaux cyclohexyle, phényle ou naphtyle éventuellement substitués,
- $R_{a1}$, $R_{a2}$, $R_{a3}$ et/ou $R_{b1}$, $R_{b2}$, $R_{b3}$ peuvent former ensemble et avec l'atome de carbone qui les porte un cycle benzénique ou naphtalénique éventuellement substitué.

Parmi tous les composés cétoniques répondant à la formule (IIa), on fait appel tout particulièrement aux composés cétoniques répondant à la formule (IIb) suivante :

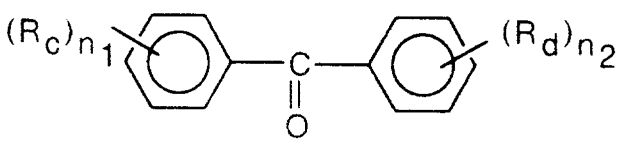

(IIb)

dans ladite formule (IIb) :

- $R_c$ et $R_d$ identiques ou différents représentent un atome d'hydrogène ou un substituant, de préférence un groupe électro-donneur,
- $n_1$, $n_2$ identiques ou différents est un nombre égal à 0, 1, 2 ou 3,

Le substituant est choisi de telle sorte qu'il ne réagisse pas dans les conditions d'acidité de l'invention. Il s'agit préférentiellement d'un groupe électro-donneur.
On entend par "groupe électro-donneur", un groupe tel que défini par H.C. BROWN dans l'ouvrage de Jerry MARCH - Advanced Organic Chemistry, chapitre 9, pages 243 et 244 (1985).
Des exemples de substituants convenant bien à l'invention sont les suivants :

- les radicaux alkyle linéaires ou ramifiés ayant de 1 à 4 atomes de carbone,
- le radical phényle,
- les radicaux alkoxy $R_{10}$ - O dans lesquels $R_{10}$ représente un radical alkyle linéaire ou ramifié ayant de 1 à 4 atomes de carbone ou le radical phényle,
- le groupe hydroxyle,
- l'atome de fluor.

Comme exemples de composés cétoniques particulièrement adaptés à l'invention, on peut citer tout particulièrement les composés cétoniques répondant à la formule générale (IIb) dans laquelle $R_c$ et $R_d$, identiques ou différents représentent un atome d'hydrogène ou un substituant tel que précité, de préférence en position 4,4' et $n_1$, $n_2$ identiques ou différents sont égaux à 0 ou 1.
On fait appel préférentiellement aux composés cétoniques répondant à la formule (IIb) dans laquelle $R_c$ et $R_d$ identiques ou différents représentent un atome d'hydrogène ; un radical méthyle, éthyle, tert-butyle, phényle ; un radical méthoxy ou éthoxy ; un groupe hydroxyle, de préférence en position 3,3' ou 4,4'.
Comme exemples spécifiques de cétones qui peuvent être utilisées dans le procédé de l'invention, on peut citer plus particulièrement :

- la benzophénone
- la méthyl-2 benzophénone
- la diméthyl-2,4 benzophénone
- la diméthyl-4,4' benzophénone
- la diméthyl-2,2' benzophénone
- la diméthoxy-4,4' benzophénone
- l'hydroxy-4 benzophénone
- la dihydroxy-4-4' benzophénone
- le benzoyl-4 biphényle

Selon le procédé de l'invention, on fait réagir, le composé phénolique de formule (I) avec le composé cétonique de formule générale (II), en présence d'un catalyseur acide.

Le catalyseur convenant à l'invention est choisi de telle sorte qu'il présente une fonction d'acidité - Ho comprise entre 5 et 20, de préférence entre 10 et 15.

Le catalyseur intervenant dans le procédé de l'invention est un catalyseur acide. Il peut s'agir d'une catalyse homogène ou hétérogène.

On fait appel à un acide protonique fort.

Par acide fort, on désigne dans la présente invention, un acide présentant un pKa dans l'eau inférieur à - 0,1 et, de préférence, inférieur à - 1,0.

Comme exemples non limitatifs de tels acides forts, on peut citer les acides halogénés tels que l'acide chlorhydrique, l'acide bromhydrique, l'acide fluorhydrique, les oxyacides halogénés ou non tels que l'acide sulfurique, l'acide pyrosulfurique, l'acide perchlorique, les acides halogénosulfoniques tels que l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

Parmi ces acides, on utilisera de préférence l'acide fluorhydrique, l'acide perchlorique, l'acide trifluorométhanesulfonique, l'acide paratoluènesulfonique, l'acide chlorosulfonique, l'acide fluorosulfonique, l'acide méthanesulfonique, l'acide benzène sulfonique.

On choisit, tout particulièrement, l'acide fluorhydrique, l'acide perchlorique, l'acide trifluorométhanesulfonique ou l'acide méthanesulfonique.

Comme autres exemples de catalyseurs acides protoniques, on peut citer les résines sulfoniques.

Ainsi, on peut mettre en oeuvre dans le procédé de l'invention, les résines sulfoniques existant sur le marché, résines commercialisées sous différentes dénominations commerciales. On peut citer, entre autres, les résines suivantes : TEMEX 50, AMBERLYST 15, AMBERLYST 35, AMBERLYST 36, DOWEX 50W.

Les résines précitées qui conviennent bien à la présente invention sont constituées d'un squelette polystyrénique qui porte des groupes fonctionnels qui sont des groupes sulfoniques.

Le squelette polystyrénique est obtenu par polymérisation du styrène et du divinylbenzène, sous l'influence d'un catalyseur d'activation, le plus souvent un peroxyde organique, ce qui conduit à un polystyrène réticulé. La polymérisation se fait le plus souvent en suspension et l'on obtient des billes ou des granules de polymère. Ceux-ci sont traités par de l'acide sulfurique ou sulfochlorique concentré. On obtient un copolymère styrène-divinylbenzène sulfoné.

Il est également possible de faire appel à des résines sulfoniques qui sont des copolymères phénol-formol et qui portent sur le noyau aromatique un groupe méthylène sulfonique. Comme exemples desdites résines, on peut mentionner entre autres, la résine commercialisée sous la dénomination DUOLITE ARC 9359.

D'autres résines sont également disponibles sur le marché et l'on peut citer les résines perfluorées porteuses de groupes sulfoniques et, plus particulièrement le NAFION qui présente la structure générale donnée ci-après :

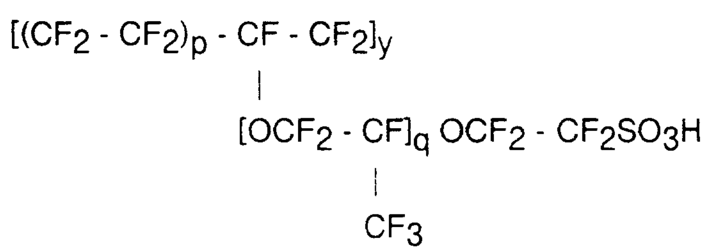

dans ladite formule, q est un nombre entier égal à 1,2,3,---, p est compris entre 5 et 13,5 et y est égal à environ 1000. Le NAFION est préparé à partir d'un copolymère de tétrafluoroéthylène et de perfluoro[2-(fluorosulfonyléthoxy)-propyl] vinyl éther.

Les résines en cause peuvent être du type gel ou du type macroréticulé. Elles sont mises en oeuvre sous la forme acide.

De nombreuses résines sont des produits disponibles sur le marché sous forme sèche ou humide. L'une ou l'autre des formes peuvent être utilisées dans le procédé de l'invention.

Elles se présentent habituellement sous forme de particules sensiblement sphériques ayant un diamètre variant de 0,3 à 1,5 mm, de préférence entre 0,5 et 1,2 mm.

Les résines précitées sont mises en oeuvre préférentiellement dans le procédé de l'invention et plus préférentiellement les résines ayant un squelette polystyrénique. Toutefois, l'invention n'exclut pas la mise en oeuvre de résines ayant un squelette d'une autre nature dans la mesure où elle porte les groupes sulfoniques adéquates.

La proportion de groupes sulfoniques par rapport à la masse polymérique peut être variable et l'on en tiendra compte lors de la détermination de la quantité de polymère à mettre en oeuvre.

La concentration en sites acides du polymère sulfonique varie avantageusement entre 1 et 10 milliéquivalents d'ions $H^+$ par gramme de polymère sec, et, de préférence, entre 2 et 7 milliéquivalents d'ions $H^+$ par gramme de polymère sec.

On assimilera également dans ce qui suit aux acides protoniques forts, les formes acides des oxydes mixtes, des argiles et des zéolithes.

Comme exemples de catalyseurs à propriétés acides, on peut citer, tout particulièrement les combinaisons d'oxydes métalliques et métalloïdiques telles que : silice-alumine, silice-$Ga_2O_3$,silice-$B_2O_3$.

Un autre type de catalyseurs minéraux acides convenant tout à fait bien à la mise en oeuvre du procédé de l'invention sont les argiles acides.

Pour préparer les argiles acides, on part préférentiellement des argiles naturelles présentant une structure dite "TOT" ou tétraèdre-octaèdre-tétraèdre.

Les argiles TOT se présentent sous forme de feuillets élémentaires comprenant deux couches de tétraèdres d'atomes d'oxygène dans lesquels sont inclus les atomes de silicium séparées par une couche d'octaèdres d'atomes d'oxygène dans lesquels est inclus le métal M de type $MO_4(OH)_2$ ou M est un cation divalent ou trivalent.

Lorsque tous les tétraèdres sont occupés par des ions $Si^{4+}$, la neutralité électrique du feuillet peut être mesurée de deux façons, selon la charge du cation octaédrique :

- s'il est divalent ($Mg^{2+}$, $Fe^{2+}$,...), toutes les cavités octaédriques sont occupées ; le feuillet est alors dit trioctaédrique,
- s'il est trivalent ($Al^{3+}$, $Fe^{3+}$,...), deux cavités octaédriques sur trois sont occupées et le feuillet est dioctaédrique.

Mais de nombreuses substitutions sont possibles, aussi bien dans la couche tétraédrique que dans la couche octaédrique. Celles-ci peuvent entraîner un déficit de charge du feuillet et la neutralité du cristal est alors réalisée par insertion de cations compensateurs entre les feuillets.

On trouve parmi les argiles TOT, les trois classes suivantes : les smectites, les vermiculites et les micas.

Parmi les argiles, la classe utilisée préférentiellement selon l'invention est celle des smectites.

Les smectites sont classifiées selon la nature du métal M (aluminium, magnésium, fer, lithium), la nature du cation compensateur (sodium, potassium, calcium) et la nature de la substitution tétraédrique ou octaédrique.

On peut ainsi citer parmi la classe des smectites :

- les montmorillonites de formule $Si_4(Al_{2-y}Mg_y)O_{10}(OH)_2,M^+{}_y$
- les beidellites de formule $(Si_{4-x}Al_x)Al_2O_{10}(OH)_2,M^+{}_x$
- les nontronites de formule $(Si_{4-x}Al_x)Fe_2O_{10}(OH)_2,M^+{}_x$
- les hectorites de formule $Si_4(Mg_{3-y}Li_y)O_{10}(OH)_2,M^+{}_y$
- les stévensites de formule $Si_4(Mg_{3-y})O_{10}(OH_2),M^+{}_{2y}$
- les saponites de formule $(Si_{4-x}Al_x)Mg_3O_{10}(OH_2),M^+{}_x$

On préfère encore tout particulièrement dans le cadre de la présente invention utiliser les montmorillonites.

On peut utiliser les argiles commerciales déjà acides telles que notamment : les argiles suivantes : KSF, TONSIL OPTIMUM FF et K 10 vendues par Süd Chemie.

On peut également traiter les argiles commerciales, déjà acides ou non par une solution aqueuse d'un acide. La concentration de la solution aqueuse en acide est variable, cependant elle devra avoir un pH supérieur ou égal à 2 de façon à ne pas détruire l'argile et elle devra contenir une quantité d'ions $H^+$ exprimé en milliéquivalents correspondant à au moins la capacité d'échange de large. On définit la capacité d'échange d'une argile comme le nombre de cations monovalents, exprimé en milliéquivalents que peuvent échanger 1 g d'échantillon.

Cette capacité d'échange (ou charge par demi-maille) varie pour les smectites entre 0,2 et 0,6 et pour les vermiculites entre 0,6 et 0,9. Il est préférable donc dans le cadre de la présente invention d'utiliser une solution acide contenant autant d'équivalents $H^+$ qu'il y aura de cations à échanger dans large, donc contenant au moins 0,6 et de préférence au moins 1 milliéquivalent acide pour 1 g d'argile.

On peut éventuellement, pour améliorer la surface d'échange de large, la traiter ensuite avec un alcool tel que le méthanol, puis la sécher.

Conviennent également à la mise en oeuvre du procédé de l'invention, les argiles de type pontée.

On entend par argile pontée, des argiles entre les feuillets desquelles ont été introduits des ponts ou piliers qui maintiennent un espacement basal. L'espacement basal (ou distance interfoliaire) est la somme de l'épaisseur d'un feuillet de l'argile et de l'espacement interfoliaire.

Comme exemples d'espèces minérales créant les piliers ou ponts, on peut citer celles dérivées des éléments suivants : aluminium, nickel, cobalt, vanadium, molybdène, rhénium, fer, cuivre, ruthénium, chrome, lanthane, cérium, titane, bore, gallium, zirconium, niobium, tantale, silicium.

Les argiles pontées mises en oeuvre préférentiellement dans le procédé de l'invention sont les argiles pontées par des piliers d'oxyde d'aluminium, de zirconium, de chrome, de silicium et de cérium.

Les dites argiles sont préparées selon les procédés décrits dans la littérature.

On peut se référer, entre autres, pour la préparation d'argiles pontées à l'aluminium à FR-A 2 563 446 et à FR-A 2 656 298 ; d'argiles pontées au titane à FR-A 2 669 016 et d'argiles pontées au cérium à la demande de brevet française n°91/04409.

Parmi les argiles pontées, on fait appel tout particulièrement aux argiles de type smectite. Parmi ces smectites, on peut citer à titre d'exemples non limitatifs, les beidellites, en particulier les beidellites synthétiques et les montmorillonites.

Les argiles pontées peuvent être obtenues par un procédé comportant les étapes suivantes :

a) traitement d'une argile naturelle ou synthétique, sous forme d'une suspension aqueuse, par une solution aqueuse contenant au moins un hydroxyde d'au moins un métal,
b) l'élimination de l'excès d'hydroxyde de métal n'ayant pas réagi sur l'argile ; ladite élimination et séchage de l'argile traitée,
c) le traitement thermique de l'argile traitée ; ledit traitement conduisant à l'obtention de l'argile pontée.

Pour la préparation des argiles pontées utilisables dans le cadre de l'invention, on peut également mettre en oeuvre que les étapes a) et b).

Le pontage des argiles peut être effectué à l'aide des hydroxydes des métaux précités.

De préférence, on utilise comme catalyseur dans le procédé de l'invention, une beidellite ou une montmorillonite pontée à l'aide d'hydroxyde d'aluminium, par exemple selon le procédé décrit dans FR-A 2 563 446.

Comme exemples de catalyseurs convenant également à la présente invention, on peut citer les zéolithes naturelles telles que par exemple : la chabazite, la clinoptilolite, l'érionite, la mordénite, la phillipsite, l'offrétite.

Conviennent tout à fait bien à la mise en oeuvre de l'invention, les zéolithes synthétiques telles que la zéolithe ZSM-5, la zéolithe Y, la ferrierite ; la zéolithe X de type faujasite, la zéolithe de type L, la mordénite, la zéolithe ZSM-11 ; la mazzite, l'offrétite. Quelle que soit la zéolithe, on fait un traitement qui la rend acide.

On fait appel préférentiellement aux zéolithes synthétiques et plus particulièrement aux zéolithes commerciales qui sont sous les formes suivantes :

- des zéolithes sous forme acide telles que les zéolithes ZSM-5 ou silicalite d'aluminium de rapport molaire Si/Al de 10 à 500 ; les zéolithes Y en particulier les zéolithes obtenues après traitement de désalumination (par exemple hydrotraitement, lavage à l'aide d'acide chlorhydrique ou traitement par $SiCl_4$) et l'on peut citer plus particulièrement les zéolithes US-Y de rapport molaire Si/Al supérieur à 3, de préférence compris entre 6 et 60 ; les zéolithes telles que la ferrierite de rapport molaire Si/Al de 5 à 10,
- des zéolithes sous forme sodique ou potassique échangeable telles que la zéolithe X de type faujasite de rapport molaire Si/Al de 1 à 1,5, la zéolithe de type L de rapport molaire Si/Al de 3 à 3,5, la mordénite de rapport molaire Si/Al de 5 à 6, la zéolithe ZSM-11 de rapport molaire Si/Al de 5 à 30,
- des zéolithes à la fois sous forme acide ou sous forme sodique ou potassique échangeable telles que la mazzite de rapport molaire Si/Al de 3,4, l'offrétite de rapport molaire Si/Al de 4.

Conformément au procédé de l'invention, la zéolithe mise en oeuvre dans le procédé de l'invention est une zéolithe acidifiée.

Pour acidifier la zéolithe si nécessaire, on fait appel aux traitements classiques.

Ainsi, on peut échanger les cations alcalins en soumettant la zéolithe à un traitement réalisé avec de l'ammoniaque conduisant ainsi à un échange du cation alcalin par un ion ammonium puis à calciner la zéolithe échangée afin de décomposer thermiquement le cation ammonium et le remplacer par un ion $H^+$.

La quantité d'ammoniaque à mettre en oeuvre est au moins égale à la quantité nécessaire pour échanger tous les cations alcalins en ions $NH_4^+$.

On met donc au moins en jeu de $5.10^{-3}$ à $10^{-5}$ mole d'ammoniaque par gramme de zéolithe.

La réaction d'échange du cation échangeable par $NH_4^+$ est effectuée à une température qui se situe entre la température ambiante et la température de reflux du milieu réactionnel. L'opération dure quelques heures et peut être répétée.

Les zéolithes peuvent être également acidifiées en soumettant celles-ci à un traitement acide classique. Ce traitement peut être effectué par addition d'un acide tel que notamment l'acide chlorhydrique, l'acide sulfurique, l'acide nitrique, l'acide perchlorique, l'acide phosphorique et l'acide trifluorométhanesulfonique.

Selon un mode préférentiel de mise en oeuvre, la zéolithe est acidifiée par passage d'un volume d'acide présentant une normalité comprise entre 0,1 et 2 N par gramme de zéolithe comprise entre 10 ml/g et 100ml/g. Ce passage peut être réalisé en une seule étape ou de préférence en plusieurs étapes successives.

On ne sortira pas du cadre de la présente invention, à faire appel à une zéolithe acide modifiée par exemple en faisant appel à des zéolithes de type ZSM-5 et ZSM-11 dans lesquelles une partie de l'aluminium est remplacé par un élément tel que fer, gallium, bore, indium, chrome, scandium, cobalt, nickel, beryllium, zinc, cuivre, antimoine, arsenic, vanadium ou leur mélange.

Comme exemples d'autres catalyseurs solides à propriétés acides convenant à la mise en oeuvre du procédé de l'invention, on peut citer les phosphates tels que notamment les phosphates de bore, d'aluminium ou de gallium.

On peut en particulier faire appel aux phosphates lamellaires de métaux tétravalents répondant à la formule $\alpha$- $M(HPO_4)_2$, $pH_2O$ dans laquelle M représente un métal tétravalent choisi parmi le titane, le germanium, le zirconium ou l'étain et p est un nombre inférieur à 2. Lesdits composés sont décrits dans la littérature et l'on peut se référer entre autres aux travaux de ALBERTI et al - J. of Inorg. Nucl. Chem. <u>40</u>, p. 1113 (1978).

On peut également mettre en oeuvre, des phosphonates lamellaires de métaux tétravalents et tout particulièrement les phosphonates lamellaires de zirconium (US-A 4 436 899).

Conviennent également à invention, les oxydes rendus acides par un traitement. On peut citer les oxydes sulfatés, chlorés ou fluorés. Ceux qui sont mis en oeuvre préférentiellement sont les oxydes sulfatés de titane, de zirconium ou de fer. Ces oxydes sont décrits dans la littérature et l'on peut se référer entre autres à EP-A 0 397 553.

Les modes préférés de réalisation de l'invention consistent à faire appel aux catalyseurs acides suivants : l'acide fluorhydrique, l'acide perchlorique, l'acide trifluorométhanesulfonique, l'acide méthanesulfonique et les résines sulfoniques de type NAFION.

Pour ce qui est du composé soufré intervenant éventuellement dans le procédé de l'invention, on peut faire appel plus particulièrement aux composés suivants :

. composés minéraux soufrés :

- les halogénures de soufre, de préférence le monochlorure de soufre ou le bichlorure de soufre,
- les thiosulfates d'ammonium, de métal alcalin ou alcalino-terreux, de préférence le thiosulfate d'ammonium, de sodium ou de potassium,
- l'hydrogène sulfuré ou ses précurseurs tels que, par exemple, le sulfure de calcium ou de potassium qui, ajoutes au milieu réactionnel acide, conduisent à la formation d'hydrogène sulfuré,

. composés organiques soufrés :

- les mercaptans notamment les alkylmercarptans, phénylmercaptans et phénylalkylmercaptans tels que par exemple, l'éthylmercaptan, le butylmercaptan, le 1-nonylmercaptan, le benzylmercaptan,
- les thioalcools comme par exemple, le 2-mercaptoéthanol, le 3-mercapto-2-butanol,
- les thiophénols tels que notamment, le thiophénol, l'o-thiocrésol, le m-thiocrésol, le p-thiocrésol, le thioxylénol, le p-méthylthiophénol, l'o-éthylthiophénol, le thiohydroquinone, le thionaphtol,
- les acides thioorganiques, leurs sels ou leurs esters tels que l'acide thioacétique, l'acide thiopropionique, l'acide thiolactique, l'acide thiosalicylique, l'acide 2-mercaptoéthanesulfonique, l'acide 3-mercaptopropanesulfonique, l'acide mercaptosuccinique, l'acide thioglycolique, le thioglycolate de méthyle, d'éthyle,
- les résines échangeuses de cations modifiées par réaction avec une alkyl mercaptoamine, de préférence es résines à squelette polystyrénique porteuses de groupes sulfoniques telles que précédemment définies, ayant réagi avec une alkyl mercaptoamine, de préférence celle avec un groupe amine primaire et encore plus préférentiellement celle ayant de 1 à 4 atomes de carbone, et notamment, la 2-mercaptoéthylamine, la 2-mercaptoisopropylamine, la 3-mercaptobutylamine.

Les composé soufrés choisis préférentiellement sont les alkylmercaptans et le benzylmercaptan.

Conformément au procédé de l'invention, on effectue la préparation de la p-fuchsone par réaction du composé phénolique de formule (I), et du composé cétonique de formule (II), en présence du catalyseur acide et éventuellement d'un composé soufré ionisable.

La quantité de composé phénolique de formule (I) mis en oeuvre et exprimée par rapport au composé cétonique de formule (II) est généralement au moins égale à la quantité stoechiométrique. On choisit avantageusement un rapport molaire entre le composé phénolique de formule (I) et le composé cétonique de formule (II) compris entre 1 et 20, de préférence entre 1 et 10.

Pour ce qui est du catalyseur acide, la quantité d'acide exprimée par le rapport d'équivalents de protons au nombre de moles de composé cétonique de formule (II) peut varier entre $1.10^{-3}$ et 1,0, de préférence entre $5.10^{-3}$ et 0,5.

Il est également possible d'utiliser l'acide en tant que milieu réactionnel. On peut citer notamment le cas de l'acide fluorhydrique et de l'acide trifluorométhanesulfonique. Ainsi, le rapport précité peut être supérieur à 1,0 et être très élevé et dépassé 20. D'une manière préférée, il est compris entre 5,0 et 10.

S'agissant d'un catalyseur hétérogène solide, la quantité à mettre en oeuvre est déterminée de telle sorte que le catalyseur représente en poids par rapport au composé phénolique de formule (I) engagé, de 0,1 à 20 %, de préférence de 0,5 à 10 %. Cependant si l'on réalise le procédé en continu, par exemple en faisant réagir un mélange de composé phénolique de formule (I) et de composé cétonique de formule (II), sur un lit fixe de catalyseur, ces rapports catalyseur/composé cétonique de formule (II) n'ont pas de sens et à un instant donné, on pourra avoir un excès pondéral de catalyseur par rapport au composé cétonique de formule (II).

La quantité de composé soufré mis en oeuvre exprimée par rapport au composé cétonique de formule (II) est choisie avantageusement de telle sorte que le rapport molaire entre le composé soufré et le composé cétonique de formule (II) soit compris entre 0,001 et 1,0, de préférence entre 0,005 et 0,20.

La préparation de la fuchsone conformément au procédé de l'invention, est réalisée à une température qui peut être comprise entre 45°C et 200°C.

Une variante préférée du procédé de l'invention consiste à choisir la température entre 60°C et 150°C.

La réaction est conduite avantageusement sous pression atmosphérique.

La préparation de la fuchsone conformément au procédé de l'invention peut être également conduite, en présence d'un solvant organique.

L'un des intérêts de la mise en oeuvre d'un solvant organique est la possiblité d'éliminer l'eau du milieu réactionnel, par distillation azéotropique.

On peut faire appel à tout solvant organique inerte dans les conditions réactionnelles. Comme exemples de solvants organiques convenant bien à la présente invention, on peut mentionner entre autres :

- les hydrocarbures aliphatiques et/ou aromatiques et plus particulièrement les paraffines tels que notamment, l'hexane, l'heptane, l'octane, le nonane, le décane, le undécane, le dodécane ou le tétradécane, le cyclohexane, le méthylcyclohexane ; les hydrocarbures aromatiques comme notamment les xylènes, le cumène, les coupes pétrolières constituées de mélange d'alkylbenzènes notamment les coupes de type Solvesso®,
- les hydrocarbures halogénés aliphatiques ou aromatiques, et l'on peut mentionner : les hydrocarbures perchlorés tels que notamment le tétrachlorure de carbone, le tétrachloroéthylène, l'hexachloroéthane, l'hexachloropropène, les hydrocarbures partiellement chlorés tels que le chlorure de méthylène, le dichloroéthane, le tétrachloroéthane, le trichloroéthylène, le 1-chlorobutane, le 1,2-dichlorobutane ; le mono-chlorobenzène, le 1,2-dichlorobenzène, le 1,3-dichlorobenzène, le 1,4-dichlorobenzène ou des mélanges de différents chlorobenzènes ; le monobromobenzène ou des mélanges de monobromobenzène avec un ou plusieurs dibromobenzènes.

On peut également mettre en oeuvre un mélange de solvants organiques.

La concentration des réactifs dans le solvant organique peut varier largement. La concentration de la cétone non énolisable dans le solvant organique peut varier, par exemple, entre 0,1 et 2 mol/litre, de préférence, entre 0,2 et 1,0 mol/litre.

D'un point de vue pratique, le procédé selon l'invention est simple à mettre en oeuvre de façon continue ou discontinue.

Les différents réactifs peuvent être introduits dans n'importe quel ordre. D'une manière préférée, on choisit l'ordre des réactifs suivants : on introduit le composé phénolique de formule (I), le composé cétonique de formule (II) et le catalyseur acide et éventuellement un composé soufré ionisable. On peut également additionner un solvant organique.

On porte le milieu réactionnel à la température désirée, tout en maintenant le milieu réactionnel sous agitation.

Au cours de la réaction, il y a formation d'eau dans le milieu réactionnel. Une variante préférée de l'invention consiste à limiter la concentration de l'eau dans le milieu réactionnel, en éliminant celle-ci au fur et à mesure de sa formation, par tout moyen connu, notamment par distillation azéotropique.

En fin de réaction, on récupère la fuchsone formée à partir du milieu réactionnel.

On commence par séparer le catalyseur. S'il s'agit d'une catalyse homogène, on lave la masse réactionnelle à l'eau et l'on additionne un solvant organique convenant pour extraire les réactifs n'ayant pas réagi et la fuchsone formée. Comme exemples de solvants organiques, on peut citer notamment l'acétate d'éthyle, le dichlorométhane. Le catalyseur acide passe en phase aqueuse et l'on récupère en phase organique, la fuchsone formée et éventuellement les réactifs qui n'ont pas été transformés.

Dans le cas d'un catalyseur acide hétérogène, on lave le catalyseur acide solide à l'eau en présence d'un solvant organique qui extrait les réactifs n'ayant pas réagi et la fuchsone formée, on sépare le catalyseur selon les techniques classiques de séparation solide-liquide, de préférence par filtration puis on sépare la phase organique.

On peut récupérer la fuchsone à partir de la phase organique, par chromatographie liquide préparative sur colonne de silice.

Il est également possible de séparer la fuchsone, du composé phénolique non transformé et du composé cétonique de formule (II), par les moyens usuels, notamment, par cristallisation.

En ce qui concerne le composé soufré, celui-ci peut selon sa nature, se retrouver, soit dans la phase aqueuse, soit dans la phase organique. Il peut être récupéré selon les procédés habituels de séparation, notamment par distillation.

Le procédé, objet de la présente invention, présente de nombreux avantages. Il fait appel à des matières premières aisément disponibles et permet d'obtenir la fuchsone avec une très bonne sélectivité.

Le perfectionnement qui consiste à ajouter un composé soufré ionisable permet d'augmenter la vitesse réactionnelle de formation de la fuchsone, d'une manière appréciable.

Le présent procédé permet d'obtenir une fuchsone que l'on peut symboliser par la formule générale suivante (III) :

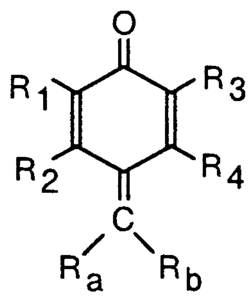

(III)

. dans ladite formule (III) les différents symboles $R_1$, $R_2$, $R_3$, $R_4$, $R_a$ et $R_b$ ont la ont la signification donnée précédemment, à savoir :

- $R_a$ et $R_b$, identiques ou différents sont des radicaux hydrocarbonés ayant chacun de 3 à 30 atomes de carbone ; les atomes de carbone de chaque radical $R_a$ et $R_b$ en position $\alpha$ par rapport à l'atome de carbone qui les porte, étant des carbones tertiaires,
- $R_1$, $R_2$, $R_3$ et $R_4$, identiques ou différents représentent un atome d'hydrogène ou un substituant quelconque,
- deux groupes $R_1$ et $R_2$ et/ou $R_3$ et $R_4$ placés sur deux atomes de carbone vicinaux peuvent former ensemble et avec les atomes de carbone qui les portent un cycle, de préférence un carbocycle insaturé ou aromatique ayant de 4 à 7 atomes de carbone.

Il convient tout particulièrement bien à la préparation de diphénylfuchsone à partir de phénol et de benzophénone.

Le procédé de l'invention permet d'accéder à une fuchsone de formule (III) mais il est à noter qu'une partie de la fuchsone formée peut se trouver sous une forme hydratée que l'on nomme carbinol et qui peut être représentée par la formule suivante (IV) :

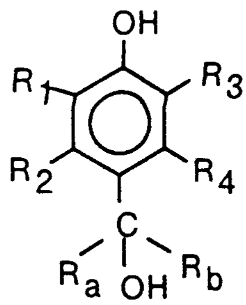 (IV)

dans ladite formule (IV), les différents symboles $R_1$, $R_2$, $R_3$, $R_4$, $R_a$ et $R_b$ ont les significations données précédemment.

Le rapport molaire fuchsone/carbinol varie avec la quantité d'eau mise en oeuvre lors du traitement de la masse réactionnelle et avec le pH du milieu. A titre d'exemples, on précise que ce rapport peut aller de 1 à 2.

On donne ci-après des exemples de réalisation de l'invention.

Les exemples 1 à 49 qui suivent, illustrent l'invention sans toutefois la limiter.

<u>EXEMPLES 1 à 49</u>

Les exemples 1 à 22 concernent la préparation de p-fuchsone.

Les exemples 23 à 49 illustrent la variante d'exécution du procédé de l'invention avec mise en oeuvre d'un composé soufré ionisable.

Dans les exemples, les abréviations suivantes signifient :

$$TT_{CETONE} = \frac{\text{nombre de moles de cétone transformées}}{\text{nombre de moles de cétone introduites}}\ \%$$

$$RR_{FUCHSONE} = \frac{\text{nombre de moles de fuchsone formées}}{\text{nombre de moles de cétone introduites}}\ \%$$

$$RT_{FUCHSONE} = \frac{\text{nombre de moles de fuchsone formées}}{\text{nombre de moles de cétone transformées}}\ \%$$

En ce qui concerne l'expression des résultats, les RR et RT sont donnés sans discerner la fuchsone de sa forme hydratée, le carbinol. On appelle donc fuchsone, le mélange de fuchsone et de son carbinol. Comme mentionné précédemment, la quantité de fuchsone hydratée dépend du traitement réactionnel : ce rapport est généralement voisin de 1,5.

<u>Exemple 1</u>

Dans un ballon de verre de 100 ml muni d'une agitation centrale, d'un réfrigérant, d'une ampoule de coulée et d'un thermomètre, on charge :

- 19,8 g (0,2 mol) de phénol
- 18,2 g (0,1 mol) de benzophénone
- 7,5 g (0,05 mol) d'acide trifluorométhanesulfonique $CF_3SO_3H$

On porte le mélange réactionnel à la température réactionnelle choisie 70°C, tout en le maintenant sous agitation de 1200 tours/mn.

Au bout de 19 heures, on refroidit alors le mélange réactionnel et l'on effectue le dosage des produits de la réaction : la diphénylfuchsone obtenue et la benzophénone résiduelle sont dosées par chromatographie en phase gazeuse.

Les résultats obtenus sont les suivants :

- $RR_{FUCHSONE}$ = 26,2 %

- TT $_{BENZOPHENONE}$ = 28,6 %
- RT $_{FUCHSONE}$ = 91,5 %

Exemple 2

Dans cet exemple, on reproduit l'exemple 1 sauf que la température réactionnelle est de 80°C au lieu de 70°C.
Au bout de 4 heures de réaction, les résultats obtenus sont les suivants :

- RR $_{FUCHSONE}$ = 20,8 %
- TT $_{BENZOPHENONE}$ = 20,8 %
- RT $_{FUCHSONE}$ = 100 %

Au bout de 5 heures de réaction, les résultats obtenus sont les suivants :

- RR $_{FUCHSONE}$ = 22,6 %
- TT $_{BENZOPHENONE}$ = 22,6 %
- RT $_{FUCHSONE}$ = 100 %

Exemple 3

Dans un appareillage tel que décrit dans l'exemple 1, on charge :

- 19,8 g (0,20 mol) de phénol
- 18,2 g (0,10 mol) de benzophénone
- 0,75 g (0,005 mol) d'acide trifluorométhanesulfonique $CF_3SO_3H$

On porte le mélange réactionnel à la température de 80°C, tout en le maintenant sous agitation de 1200 tours/mn.
Dans cet exemple, on reproduit l'exemple 1 sauf que la température réactionnelle est de 150°C au lieu de 70°C.
Au bout d'1 heure de réaction, les résultats obtenus sont les suivants :

- RR $_{FUCHSONE}$ = 3,8 %
- TT $_{BENZOPHENONE}$ = 4,6 %
- RT $_{FUCHSONE}$ = 83 %

Au bout de 6 heures de réaction, les résultats obtenus sont les suivants :

- RR $_{FUCHSONE}$ = 3,8 %
- TT $_{BENZOPHENONE}$ = 6,5 %
- RT $_{FUCHSONE}$ = 58,5 %

Exemple 4

Dans un appareillage tel que décrit dans l'exemple 1, on charge :

- 9,4 g (0,10 mol) de phénol
- 18,2 g (0,10 mol) de benzophénone
- 7,5 g (0,05 mol) d'acide trifluorométhanesulfonique $CF_3SO_3H$

On porte le mélange réactionnel à la température de 80°C, tout en le maintenant sous agitation de 1200 tours/mn.
Au bout de 4 heures de réaction, les résultats obtenus sont les suivants :

- RR $_{FUCHSONE}$ = 17 %
- TT $_{BENZOPHENONE}$ = 17 %
- RT $_{FUCHSONE}$ = 100 %

On ajoute alors 0,05 mol d'acide trifluorométhanesulfonique $CF_3SO_3H$.

Au bout de 6 heures 45, les résultats obtenus sont les suivants :

- RR $_{FUCHSONE}$ = 29 %
- TT $_{BENZOPHENONE}$ = 29 %
- RT $_{FUCHSONE}$ = 100 %

Exemple 5

On reproduit l'exemple 1 à la seule différence que l'on met en oeuvre de l'acide perchlorique à 70% dans une même quantité.

La température réactionnelle est aussi de 70°C.

Au bout de 28 heures de réaction, les résultats obtenus sont les suivants :

- RR $_{FUCHSONE}$ = 5,9 %
- TT $_{BENZOPHENONE}$ = 6,0 %
- RT $_{FUCHSONE}$ = 98 %

Exemple 6

Dans un appareillage tel que décrit dans l'exemple 1, on charge :

- 19,8 g (0,2 mol) de phénol
- 18,2 g (0,1 mol) de benzophénone
- 4,8 g (0,05 mol) d'acide méthanesulfonique

On porte le mélange réactionnel à la température réactionnelle choisie de 150°C, tout en le maintenant sous agitation de 1200 tours/mn.

Au bout de 5 heures de réaction, les résultats obtenus sont les suivants :

- RR $_{FUCHSONE}$ = 8 %
- TT $_{BENZOPHENONE}$ = 15 %
- RT $_{FUCHSONE}$ = 53 %

Exemple 7

On reproduit l'exemple précédent mais en remplacant l'acide méthanesulfonique par l'acide benzènesulfonique.

La température réactionnelle choisie est de 120°C.

Au bout de 4 heures 30 minutes de réaction, les résultats obtenus sont les suivants :

- RR $_{FUCHSONE}$ = 1,5 %
- TT $_{BENZOPHENONE}$ = 2,4 %
- RT $_{FUCHSONE}$ =63 %

Exemple 8

On reproduit l'exemple 6 à la seule différence que l'on remplace l'acide méthanesulfonique par l'acide pyrophosphorique.

Au bout de 3 heures de réaction, les résultats obtenus sont les suivants :

- RR $_{FUCHSONE}$ = 1 %
- TT $_{BENZOPHENONE}$ = 1 %
- RT $_{FUCHSONE}$ = 100 %

Exemple 9

Dans un appareillage tel que décrit dans l'exemple 1, on charge :

- 19,8 g (0,2 mol) de phénol
- 9,2 g (0,1 mol) de benzophénone
- 1,1 g (0,014 mol) d'acide bromhydrique

L'acide bromhydrique est introduit sous forme gazeuse par bullage de 1,1 g dans le mélange réactionnel, pendant 20 minutes.
La température réactionnelle choisie est de 75 °C.
Au bout de 6 heures de réaction, les résultats obtenus sont les suivants :

- $RR_{FUCHSONE}$ = 2,9 %
- $TT_{BENZOPHENONE}$ = 3,2 %
- $RT_{FUCHSONE}$ = 90,6 %

Exemple 10

On reproduit l'exemple 9 mais en substituant l'acide bromhydrique par l'acide chlorhydrique gazeux, par bullage pendant toute la durée de l'essai. On opère en présence d'1 ml d'eau.
Au bout de 16 heures de réaction, les résultats obtenus sont les suivants :

- $RR_{FUCHSONE}$ = 1 %
- $TT_{BENZOPHENONE}$ = 1 %
- $RT_{FUCHSONE}$ = 100 %

Exemple 11

Dans un réacteur métallique de 100 ml, on charge :

- 4,7 g (0,050 mol) de phénol
- 9,1 g (0,05 mol) de benzophénone
- 20 g (1,00 mol) d'acide fluorhydrique anhydre

On ferme le réacteur et l'on porte le mélange réactionnel à la température choisie de 80°C, tout en le maintenant sous agitation.
Au bout de 4 heures, le mélange réactionnel est envoyé dans 100 ml d'eau ; on extrait par le dichlorométhane. La phase organique obtenue est lavée à l'eau jusqu'à pH = 5. On sépare les phases aqueuse et organique.
On effectue ensuite les dosages de la benzophénone et de la fuchsone.
Les résultats obtenus sont les suivants :

- $RR_{FUCHSONE}$ = 31 %
- $TT_{BENZOPHENONE}$ = 36 %
- $RT_{FUCHSONE}$ = 86 %

Exemple 12

Dans cet exemple, on reproduit l'exemple 11 sauf que la température réactionnelle est de 120°C au lieu de 80°C.
Au bout de 4 heures de réaction, les résultats obtenus sont les suivants :

- $RR_{FUCHSONE}$ = 10 %
- $TT_{BENZOPHENONE}$ = 18 %
- $RT_{FUCHSONE}$ = 55,5 %

Exemple 13

Dans un appareillage tel que décrit dans l'exemple 1, on charge :

- 9,4 g (0,20 mol) de phénol
- 9,1 g (0,10 mol) de benzophénone

- 2,0 g de zéolithe US-Y

La zéolithe US-Y mise en oeuvre est une zéolithe commercialisée par la société TOSOH ayant un rapport molaire Si/Al égal à 6,0.
La température réactionnelle choisie est de 150°C.
Au bout de 5 heures de réaction, les résultats obtenus sont les suivants :

- $RR_{FUCHSONE}$ = 2 %
- $TT_{BENZOPHENONE}$ = 2 %
- $RT_{FUCHSONE}$ = 100 %

Exemple 14

On reproduit l'exemple 13 à la différence près que l'on remplace la zéolithe US-Y par une argile KO de type VOLCLAY.
Au bout de 5 heures 30 de réaction, les résultats obtenus sont les suivants :

- $RR_{FUCHSONE}$ = 2 %
- $TT_{BENZOPHENONE}$ = 2 %
- $RT_{FUCHSONE}$ = 100 %

Exemple 15

Dans un appareillage tel que décrit dans l'exemple 1, on charge :

- 0,20 mol de phénol
- 0,10 mol de benzophénone
- 0,05 mol équivalent $H^+$ d'une résine sulfonique.

La résine sulfonique mise en oeuvre est une résine perfluorée préparée à partir d'un copolymère de tétrafluoroéthylène et de perfluoro [2-(fluorosulfonyléthoxy)-propyl] vinyl éther vendue sous la dénomination commerciale de NAFION®.
La température de la réaction est de 145°C.
Au bout de 3 heures de réaction, on obtient les résultats suivants :

- $RR_{FUCHSONE}$ = 14 %
- $TT_{BENZOPHENONE}$ = 16,8 %
- $RT_{FUCHSONE}$ = 83,5 %

Exemple 16

Dans un appareillage tel que décrit dans l'exemple 1, on charge :

- 9,4 g (0,1 mol) de phénol
- 2,14 g (0,01 mol) de 4,4'-dihydroxybenzophénone
- 3,1 g (0,02 mol) d'acide trifluorométhanesulfonique $CF_3SO_3H$

La température de la réaction est de 150°C.
Au bout de 4 heures de réaction, on obtient les résultats suivants :

- $RR_{ACIDE\ ROSOLIOUE}$ = 5 %
- $TT_{CETONE}$ = 15 %
- $RT_{ACIDE\ ROSOLIOUE}$ = 33,5 %

Exemple 17

Dans un appareillage tel que décrit dans l'exemple 1, on charge :

- 21,6 g (0,2 mol) d'ortho-crésol
- 18,2 g (0,1 mol) de benzophénone
- 7,5 g (0,05 mol) d'acide trifluorométhanesulfonique $CF_3SO_3H$

La température de la réaction est de 80°C.
Au bout de 3 heures de réaction, on obtient les résultats suivants :

- $RR_{METHYLFUCHSONE}$ = 25%
- $TT_{BENZOPHENONE}$ = 26,3%
- $RT_{METHYLFUCHSONE}$ = 95 %

Exemple 18

Dans un appareillage tel que décrit dans l'exemple 1, on charge :

- 0,22 mol de phénol
- 0,02 mol de benzophénone
- 0,20 mol d'acide trifluorométhanesulfonique hydraté $CF_3SO_3H, H_2O$

La température de la réaction est de 120°C.
Au bout de 4 heures de réaction, on obtient les résultats suivants :

- $RR_{FUCHSONE}$ = 66 %
- $TT_{BENZOPHENONE}$ = 84,5 %
- $RT_{FUCHSONE}$ = 78 %

Exemple 19

Dans un appareillage tel que décrit dans l'exemple 1, on charge :

- 23,5 g (0,25 mol) de phénol
- 4,55 g (0,025 mol) de benzophenone
- 7,5 g (0,05 mol) d'acide trifluorométhanesulfonique
- du p-xylène en quantité telle que le volume total atteigne 50 ml.

On porte le mélange réactionnel à 110°C, tout en maintenant sous agitation de 1200 tours/mn.
Après 4 heures, on refroidit alors le mélange réactionnel et l'on effectue le dosage des produits de réaction.
Les résultats obtenus sont les suivants :

- $RR_{FUCHSONE}$ = 68 %
- $TT_{BENZOPHENONE}$ = 84,5 %
- $RT_{FUCHSONE}$ = 80,5 %

Exemple 20

Dans un appareillage tel que décrit dans l'exemple 1, on charge :

- 4,7 g (0,05 mol) de phénol
- 0,91 g (0,005 mol) de benzophénone
- 4,8 g (0,05 mol) d'acide méthanesulfonique
- 30 ml de méthycyclohexane

On porte le mélange réactionnel à 80°C, tout en maintenant sous agitation de 1200 tours/mn. Le milieu est biphasique.
Après 5 heures, on refroidit alors le mélange réactionnel et, dans chaque phase, l'on effectue le dosage des produits de réaction.
Les résultats obtenus sont les suivants :

- RR $_{FUCHSONE}$ = 15 %
- TT $_{BENZOPHENONE}$ = 20 %
- RT $_{FUCHSONE}$ = 75 %

Exemple 21

On opère comme dans l'exemple 20 sauf que l'on remplace le méthylcyclohexane par le même volume de cyclohexane et que l'on élimine l'eau par distillation azéotropique.
Les résultats obtenus sont les suivants :

- RR $_{FUCHSONE}$ = 24 %
- TT $_{BENZOPHENONE}$ = 28 %
- RT $_{FUCHSONE}$ = 85,5 %

Exemple 22

Dans un appareillage tel que décrit dans l'exemple 1, on charge :

- 56,4 g (0,6 mol) de phénol
- 10,92 g (0,06 mol) de benzophénone
- 46,06 g (0,48 mol) d'acide méthanesulfonique

On porte le mélange réactionnel à 100°C pendant 7 heures, tout en maintenant sous agitation de 1200 tours/mn.
On refroidit alors le mélange réactionnel puis on ajoute 50 ml d'eau et 100 ml d'éther isopropylique. On décante la phase éthérée qui est lavée 3 fois par 50 ml d'eau.
Dans la phase organique éthérée, on dose :

- FUCHSONE = 0,0347 mol (RR = 58,3 %)
- BENZOPHENONE = 0,0240 mol (TT = 59,8 %).

Exemple 23

Dans un ballon de verre de 50 ml, muni d'une agitation centrale, d'un réfrigérant, d'un thermomètre, on charge:

- 23,5 g (0,25 mol) de phénol
- 4,55 g (0,025 mol) de benzophénone
- 7,9 g (0,05 mol) d'acide benzènesulfonique
- 0,621 g (0,005 mol) de benzylmercaptan

On porte le mélange réactionnel à la température de réaction choisie, 110°C, tout en maintenant l'agitation à 1200 tours/mn.
Au bout de 4 heures, on refroidit le mélange réactionnel et l'on effectue le dosage des produits de la réaction : la diphénylfuchsone et la benzophénone résiduelle sont dosées par chromatographie en phase gazeuse.
Les résultats obtenus sont les suivants:

- RR $_{FUCHSONE}$ = 57,6 %
- TT $_{BENZOPHENONE}$ = 62 %
- RT $_{FUCHSONE}$ = 92,9 %

Le même essai effectué sans benzylmercaptan, conduit après 4 heures de réaction à 110°C, aux résultats suivants:

- RR $_{FUCHSONE}$ = 14 %
- TT $_{BENZOPHENONE}$ = 15,2%
- RT $_{FUCHSONE}$ = 92,1 %

La figure 1 représente un graphe sur lequel sont portées deux courbes de variation du rendement (RR exprimé

en %) en fonction de la durée de la réaction (exprimée en heures), l'une en présence de benzylmercaptan (B) et l'autre en l'absence de thiol (A).

On note que la présence d'un thiol permet d'accroître considérablement la vitesse de formation de la fuchsone.

Exemple 24

Dans un ballon de verre de 50 ml, muni d'une agitation centrale, d'un réfrigérant, d'un thermomètre, on charge:

- 23,5 g (0,25 mol) de phénol
- 4,55 g (0,025 mol) de benzophénone
- 7,5 g (0,05 mol) d'acide trifluoromethanesulfonique
- 0,621 g (0,005 mol) de benzylmercaptan

On porte le mélange réactionnel à la température de réaction choisie, 80°C tout en maintenant l'agitation à 1200 tours/mn.

Au bout de 4 heures, on refroidit le mélange réactionnel et l'on effectue le dosage des produits de la réaction : la diphénylfuchsone et la benzophénone résiduelle sont dosées par chromatographie en phase gazeuse.

Les résultats obtenus sont les suivants:

- $RR_{FUCHSONE}$ = 94,8 %
- $TT_{BENZOPHENONE}$ = 99,2 %
- $RT_{FUCHSONE}$ = 95,6 %

Le même essai effectué sans benzylmercaptan, conduit après 4 heures de réaction à 80°C, aux résultats suivants:

- $RR_{FUCHSONE}$ = 63,2 %
- $TT_{BENZOPHENONE}$ = 64,8%
- $RT_{FUCHSONE}$ = 97,5 %

Exemple 25

Dans un ballon de verre de 50 ml, muni d'une agitation centrale, d'un réfrigérant, d'un thermomètre, on charge:

- 23,5 g (0,25 mol) de phénol
- 4,55 g (0,025 mol) de benzophénone
- 7,9 g (0,05 mol) d'acide benzènesulfonique
- 0,801 g (0,005 mol) de 1-nonylmercaptan

On porte le mélange réactionnel à la température de réaction choisie, 110°C tout en maintenant l'agitation à 1200 tours/mn.

Au bout de 4 heures, on refroidit le mélange réactionnel et l'on effectue le dosage des produits de la réaction : la diphénylfuchsone et la benzophénone résiduelle sont dosées par chromatographie en phase gazeuse.

Les résultats obtenus sont les suivants:

- $RR_{FUCHSONE}$ = 62 %
- $TT_{BENZOPHENONE}$ =67,6 %
- $RT_{FUCHSONE}$ = 91,7 %

Le même essai effectué sans 1-nonylmercaptan, conduit après 4 heures de réaction à 110°C, aux résultats suivants:

- $RR_{FUCHSONE}$ = 14 %
- $TT_{BENZOPHENONE}$ = 15,2 %
- $RT_{FUCHSONE}$ = 92,1 %

Exemple 26

Dans un ballon de verre de 250 ml, muni d'une agitation centrale, d'un réfrigérant, d'un thermomètre, on charge:

- 94 g (1 mol) de phénol
- 18,2 g (0,1 mol) de benzophénone
- 76,8 g (0,8 mol) d'acide méthanesulfonique
- 1,8 g (0,02 mol) de 1-butanethiol

On porte le mélange réactionnel à la température de réaction choisie, 80°C tout en maintenant l'agitation à 1200 tours/mn.

Au bout de 7 heures, on refroidit le mélange réactionnel et l'on effectue le dosage des produits de la réaction : la diphénylfuchsone et la benzophénone résiduelle sont dosées par chromatographie en phase gazeuse.

Les résultats obtenus sont les suivants:

- $RR_{FUCHSONE}$ = 96,5 %
- $TT_{BENZOPHENONE}$ = 97,4 %
- $RT_{FUCHSONE}$ = 99,1 %

Exemple 27

Dans un ballon de verre de 100 ml, muni d'une agitation centrale, d'un réfrigérant, d'un thermomètre, on charge:

- 28,2 g (0,3 mol) de phénol
- 5,46 g (0,03 mol) de benzophénone
- 23,04 g (0,24 mol) d'acide méthanesulfonique
- 0,984 g (0,006 mol) de 2-mercaptoéthanesulfonate de sodium

On porte le mélange réactionnel à la température de réaction choisie, 110°C tout en maintenant l'agitation à 1200 tours/mn.

Au bout de 4 heures, on refroidit le mélange réactionnel et l'on effectue le dosage des produits de la réaction : la diphénylfuchsone et la benzophénone résiduelle sont dosées par chromatographie en phase gazeuse.

Les résultats obtenus sont les suivant :

- $RR_{FUCHSONE}$ = 83,7 %
- $TT_{BENZOPHENONE}$ = 99 %
- $RT_{FUCHSONE}$ = 84,5 %

Le même essai est effectué sans thiol. Les résultats obtenus sont les suivants :

- $RR_{FUCHSONE}$ = 46,3 %
- $TT_{BENZOPHENONE}$ = 47 %
- $RT_{FUCHSONE}$ = 98,5 %

Exemple 28

On reproduit l'exemple 27 sauf que l'on met en oeuvre 0,246 g (0,0015 mol) de 2-mercaptoéthanesulfonate de sodium au lieu de 0,984 g (0,006 mol).

Au bout de 4 heures à 110°C, les résultats obtenus sont les suivants :

- $RR_{FUCHSONE}$ = 86,3 %
- $TT_{BENZOPHENONE}$ = 91 %
- $RT_{FUCHSONE}$ = 94,8 %

Exemple 29

On reproduit l'exemple 27 sauf que l'on met en oeuvre 0,025 g (0,00015 mol) de 2-mercaptoéthanesulfonate de sodium au lieu de 0,984 g (0,006 mol).
Au bout de 4 heures à 110°C, les résultats obtenus sont les suivants :

- RR $_{FUCHSONE}$ = 65 %
- TT $_{BENZOPHENONE}$ = 65,7 %
- RT $_{FUCHSONE}$ = 98,9 %

Exemple 30

Dans un réacteur muni d'une agitation centrale, d'un réfrigérant et d'un thermomètre, on charge:

- 8,04g (0,085 mol) de phénol
- 1,55g (0,0085 mol) de benzophénone
- 1,67g (0,0085 équivalent H+) de résine BAYER K2431
- 0,212g (0,0017 mol) de benzylmercaptan

La résine BAYER K2431 est une résine macroporeuse à squelette polystyrénique portant des groupes sulfonique, avec une concentration de sites acides de 5,1 milliéquivalents d'$H^+$ par gramme de polymère sec.
On porte le mélange réactionnel à la température choisie, 145°C, tout en maintenant l'agitation à 1200 tours/mn.
Après 4 heures de réaction, la résine est séparée du mélange réactionnel par filtration sur verre fritté, lavée avec 20ml de phénol à 80°C. On réunit le filtrat et le lavage.
La résine est agitée 1 heure à température ambiante avec un mélange d'eau et d'acétate d'éthyle 50/50, puis filtrée. La phase organique est séparée par décantation.
La diphénylfuchsone et la benzophénone résiduelle contenues dans la phase phénolique et dans la phase acétate d'éthyle, sont dosées par chromatographie en phase en phase gazeuse.
Les résultats obtenus sont les suivants:

- RR $_{FUCHSONE}$ = 11,8 %
- TT $_{BENZOPHENONE}$ = 14 %
- RT $_{FUCHSONE}$ = 84,3 %

Le même essai réalisé sans benzylmercaptan, après 4 heures de réaction à 145°C, conduit aux résultats suivants:

- RR $_{FUCHSONE}$ = 6,2 %
- TT $_{BENZOPHENONE}$ = 6,2 %
- RT $_{FUCHSONE}$ = 100 %

Exemple 31

Dans un réacteur muni d'une agitation centrale, d'un réfrigérant et d'un thermomètre, on charge:

- 8,04g (0,085 mol) de phénol
- 1,55g (0,0085 mol) de benzophénone
- 1,67g (0,0085 équivalent H+) de résine BAYER K2431
- 0,274g (0,0017 mol) de 1-nonylmercaptan

On porte le mélange réactionnel à la température choisie 145°C tout en maintenant l'agitation à 1200 tours/mn.
Après 4 heures de réaction, la résine est séparée du mélange réactionnel par filtration sur verre fritté, lavée avec 20ml de phénol à 80°C. On réunit le filtrat et le lavage.
La résine est agitée 1 heure à température ambiante avec un mélange d'eau et d'acétate d'éthyle 50/50, puis filtrée. La phase organique est séparée par décantation.
La diphénylfuchsone et la benzophenone résiduelle contenues dans la phase phénolique et dans la phase

acétate d'éthyle, sont dosées par chromatographie en phase en phase gazeuse.

Les résultats obtenus sont les suivants:

- $RR_{FUCHSONE}$ = 11,6 %
- $TT_{BENZOPHENONE}$ = 17,8 %
- $RT_{FUCHSONE}$= 65,17 %

Le même essai réalisé sans 1-nonylmercaptan, après 4 heures de réaction à 145°C, conduit aux résultats suivants:

- $RR_{FUCHSONE}$ = 6,2 %
- $TT_{BENZOPHENONE}$ = 6,2 %
- $RT_{FUCHSONE}$ = 100 %

<u>Exemple 32</u>

Dans un réacteur de 50 ml muni d'une agitation centrale, d'un thermomètre, et d'un réfrigérant, on charge:

- 0,47 g (0,005 mol) de phénol
- 0,91g (0,005 mol) de benzophénone
- 8,9 g (0,05 mol) d'acide orthophosphorique
- 0,164 g (0,001 mol) de 2-mercaptoéthanesufonate de sodium

On porte le milieu réactionnel à la température choisie, 80°C en maintenant l'agitation à 1200 tours/mn.

Au bout de 5 heures de réaction, le mélange réactionnel est refroidi et l'on effectue le dosage des produits de la réaction. La diphénylfuchsone et la benzophénone résiduelle sont dosées par chromatographie liquide haute performance.

Les résultats obtenus sont les suivants:

- $RR_{FUCHSONE}$ = 29,6 %
- $TT_{BENZOPHENONE}$ = 35,6 %
- $RT_{FUCHSONE}$ = 83,1 %

Le même essai réalisé sans 2-mercaptoéthanethiol, conduit après 5 heures de réaction à 80°C aux résultats suivants:

- $RR_{FUCHSONE}$ = 4 %
- $TT_{BENZOPHENONE}$ = 4 %
- $RT_{FUCHSONE}$= 100 %

<u>Exemple 33</u>

Dans un réacteur de 50 ml muni d'une agitation centrale, d'un thermomètre, et d'un réfrigérant, on charge:

- 0,47 g (0,005 mol) de phénol
- 0,91g (0,005 mol) de benzophénone
- 8,9 g (0,05 mol) d'acide orthophosphorique
- 0,164 g (0,001 mol) de 2-mercaptoéthanesufonate de sodium

On porte le milieu réactionnel à la température choisie, 110°C en maintenant l'agitation à 1200 tours/mn.

Au bout de 5 heures de réaction le mélange réactionnel est refroidi et l'on effectue le dosage des produits de la réaction. La diphénylfuchsone et la benzophénone résiduelle sont dosées par chromatographie liquide haute performance.

Les résultats obtenus sont les suivants:

- $RR_{FUCHSONE}$ = 30,4 %
- $TT_{BENZOPHENONE}$ = 49,6 %
- $RT_{FUCHSONE}$ = 61,3 %

Le même essai réalisé sans 2-mercaptoéthanethiol, conduit après 5 heures de réaction à 110°C aux résultats suivants:

- RR $_{FUCHSONE}$ = 3 %
- TT $_{BENZOPHENONE}$ = 10 %
- RT $_{FUCHSONE}$ = 30 %

Exemples 34 à 47

Dans un ballon de verre de 100 ml, muni d'une agitation centrale, d'un réfrigérant, d'un thermomètre, on charge :

- 28,2 g (0,3 mol) de phénol
- 23,04 g (0,24 mol) d'acide méthanesulfonique.

On ajoute de la benzophénone et du 2-mercaptoéthanesulfonate de sodium dans les rapports molaires indiqués dans le tableau (I).

On porte les mélanges réactionnels aux températures choisies, tout en maintenant l'agitation à 1200 tours/mn.

En fin de réaction, on refroidit les mélanges réactionnels et l'on effectue les dosages des produits de la réaction.

Les résultats et les conditions opératoires de chaque essai sont donnés dans le tableau (I).

Tableau (I)

| **Réf.** | Réactifs (nombre de moles) | | | | T°C de réaction (°C) | Rendements | | | | Rendements | | | |
|---|---|---|---|---|---|---|---|---|---|---|---|---|---|
| | Phénol | Ph2CO | MeSO3H | Thiol | | durée heures | TT Ph2CO | RR fuchsone | RT fuchsone | durée heures | TT Ph2CO | RR fuchsone | RT fuchsone |
| 34 | 10 | 1 | 8 | 0,2 | 80 | 4 | 96,4 | 75,8 | 78,6 | 5 | 97,6 | 79,6 | 81,6 |
| 35 | 10 | 1 | 8 | 0,05 | 80 | 4 | 80 | 75 | 93,8 | 7 | 88,7 | 86 | 97 |
| 36 | 10 | 1 | 8 | 0,05 | 140 | 4 | 95 | 88 | 92,6 | - | - | - | - |
| 37 | 10 | 1,5 | 8 | 0,05 | 110 | 5 | 76,9 | 76 | 98,8 | 7 | 80,7 | 78,4 | 97,2 |
| 38 | 10 | 1 | 8 | 0,2 | 110 | 4 | 99 | 83,7 | 84,5 | - | - | - | - |
| 39 | 10 | 0,5 | 8 | 0 | 110 | 4 | 56,6 | 55,3 | 97,7 | 7 | 74 | 70,7 | 95,5 |
| 40 | 10 | 1 | 8 | 0 | 140 | 5 | 84,8 | 73 | 86,1 | 7 | 89,7 | 77,8 | 86,7 |
| 41 | 10 | 1 | 8 | 0 | 110 | 4 | 45,2 | 43,7 | 96,6 | 7 | 60,5 | 57,8 | 95,5 |
| 42 | 10 | 1 | 8 | 0 | 110 | 4 | 43,6 | 41,3 | 94,8 | 7 | 59 | 58,7 | 99,4 |
| 43 | 10 | 2 | 8 | 0 | 110 | 4 | 34 | 33,5 | 98,5 | 7 | 44 | 43,5 | 98,9 |
| 44 | 10 | 2 | 8 | 0,005 | 110 | 4 | 41 | 40,5 | 98,8 | 7 | 50 | 50 | 100 |
| 45 | 10 | 2 | 8 | 0,05 | 110 | 4 | 59,5 | 58 | 97,5 | 7 | 66,5 | 65 | 97,7 |
| 46 | 10 | 2 | 8 | 0,1 | 110 | 4 | 67 | 64,5 | 96,3 | 7 | 74,5 | 67,5 | 90,6 |
| 47 | 10 | 3 | 8 | 0 | 110 | 4 | 22,3 | 22,3 | 100 | 7 | 30 | 30 | 100 |

Exemple 48

Dans un appareillage tel que précédemment décrit, on charge :

- 4,7 g (0,05 mol) de phénol
- 0,91 g (0,005 mol) de benzophénone
- 4,8 g (0,05 mol) d'acide méthanesulfonique
- 0,164 g (0,001 mol) de 2-mercaptoéthanesulfonate de sodium
- 30 ml de cyclohexane

On chauffe au reflux pendant 5 heures en éliminant azéotropiquement l'eau formée.
On refroidit alors la solution, on ajoute 10 ml d'eau et l'on extrait par de l'acétate d'éthyle.
Les résultats obtenus sont les suivants :

- $TT_{BENZOPHENONE} = 100\ \%$
- $RR_{FUCHSONE} = 96\ \%$

Exemple 49

Dans un appareillage tel que précédemment décrit, on charge :

- 4,7 g (0,05 mol) de phénol
- 9,1 g (0,05 mol) de benzophénone
- 89 g (0,5 mol) d'acide pyrophosphorique
- 1,64 g (0,01 mol) de 2-mercaptoéthanesulfonate de sodium

On porte ce mélange à 110°C pendant 5 heures, tout en agitant à 1200 tours/mn.
On refroidit alors la masse réactionnelle et l'on dose la benzophénone résiduelle et la diphénylfuchsone formée.
Les résultats obtenus sont les suivants :

- $TT_{BENZOPHENONE} = 50\ \%$
- $RR_{FUCHSONE} = 31\ \%$

## Revendications

1. Procédé de préparation d'une p-fuchsone caractérisé par le fait qu'il consiste à faire réagir un composé phénolique présentant au moins un atome d'hydrogène en position para et un composé cétonique non énolisable, en présence d'une quantité efficace d'un acide protonique présentant une fonction d'acidité - Ho d'au moins 5 et éventuellement en présence d'une quantité efficace d'un composé soufré ionisable.

2. Procédé selon la revendication 1 caractérisé par le fait que le composé phénolique présentant au moins un atome d'hydrogène en position para est un composé phénolique répondant à la formule générale (I) :

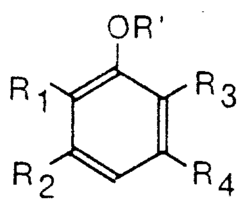
(I)

dans ladite formule (I) :

- la position en para est libre,
- $R_1$, $R_2$, $R_3$ et $R_4$, identiques ou différents représentent un atome d'hydrogène ou un substituant quelconque,
- deux groupes $R_1$ et $R_2$ et/ou $R_3$ et $R_4$ placés sur deux atomes de carbone vicinaux peuvent former ensemble et avec les atomes de carbone qui les portent un cycle,
- R' représente un atome d'hydrogène ou un radical hydrocarboné ayant de 1 à 24 atomes de carbone, qui peut être un radical aliphatique acyclique saturé ou insaturé, linéaire ou ramifié ; un radical cycloaliphatique saturé ou insaturé, monocyclique ou polycyclique ; un radical aliphatique saturé ou insaturé, linéaire ou ramifié, porteur d'un substituant cyclique.

**3.** Procédé selon l'une des revendications 1 et 2 caractérisé par le fait que le composé phénolique répond à la formule générale (I) dans laquelle :

- le radical R' représente l'un des groupes suivants :

  . un atome d'hydrogène
  . un radical alkyle linéaire ou ramifié ayant de 1 à 6 atomes de carbone, de préférence de 1 à 4 atomes de carbone,
  . un radical cyclohexyle,
  . un radical phényle,
  . un radical benzyle,

- $R_1$, $R_2$, $R_3$ et $R_4$, identiques ou différents représentent $R_0$, l'un des groupes suivants :

  . un atome d'hydrogène,
  . un radical alkyle linéaire ou ramifié, ayant de 1 à 6 atomes de carbone, de préférence de 1 à 4 atomes de carbone, tel que méthyle, éthyle, propyle, isopropyle, butyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un groupe acyle ayant de 2 à 6 atomes de carbone,
  . un radical de formule :

$$-R_5-OH$$

$$-R_5-COOR_6$$

$$-R_5-X$$

$$-R_5-CF_3$$

  dans lesdites formules, $R_5$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_6$ représente un radical alkyle linéaire ou ramifié ayant de 1 à 6 atomes de carbone ; X symbolise un atome d'halogène, de préférence un atome de chlore, de brome ou de fluor.

- $R_1$, $R_2$, $R_3$ et $R_4$, identiques ou différents représentent $R_7$, l'un des radicaux plus complexes suivants :

  . un radical carbocyclique saturé ou insaturé ayant de 4 à 7 atomes de carbone, de préférence un radical cyclohexyle,
  . un radical de for

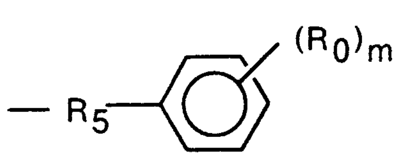

  mule dans lequel $R_5$ représente un lien valentiel ou un radical hydrocarboné divalent, linéaire ou ramifié, saturé ou insaturé ayant de 1 à 6 atomes de carbone tel que par exemple, méthylène, éthylène, propylène, isopropylène, isopropylidène et $R_0$ ayant la signification donnée précédemment et m est un nombre entier de 0 à 4,
  . un radical - $R_5$ - A - $R_8$ dans lequel $R_5$ a la signification donnée précédemment, $R_8$ représente un radical

alkyle linéaire ou ramifié ayant de 1 à 6 atomes de carbone ou un radical de formule

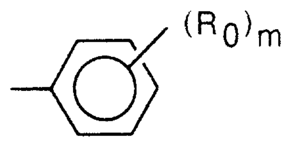

et A symbolise l'un des groupes suivants : -O-, -COO-, -OCOO-, $-SO_2-$,

- CO - N -,
|
$R_9$

dans ces formules, $R_9$ représente un atome d'hydrogène ou un radical alkyle ayant de 1 à 4 atomes de carbone, un radical cyclohexyle ou phényle.

- deux groupes $R_1$ et $R_2$ et/ou $R_3$ et $R_4$ placés sur deux atomes de carbone vicinaux peuvent former ensemble et avec les atomes de carbone qui les portent un carbocycle insaturé ou aromatique ayant de 4 à 7 atomes de carbone et, de préférence, 6 atomes de carbone,

**4.** Procédé selon l'une des revendications 1 à 3 caractérisé par le fait que le composé phénolique répond à la formule générale (I) dans laquelle :

- R' représente un atome d'hydrogène
- $R_1$, $R_2$, $R_3$ et $R_4$, identiques ou différents représentent l'un des groupes suivants :

  . un atome d'hydrogène,
  . un radical alkyle, linéaire ou ramifié ayant de 1 à 4 atomes de carbone,.
  . un radical alkoxy linéaire ou ramifié ayant de 1 à 4 atomes de carbone,
  . un groupe hydroxyle,
  . un atome d'halogène,
  . un groupe $-CF_3$
  . un radical cyclohexyle,
  . un radical phényle,

- deux groupes $R_1$ et $R_2$ et/ou $R_3$ et $R_4$ placés sur deux atomes de carbone vicinaux peuvent former ensemble et avec les atomes de carbone qui les portent un cycle benzénique.

**5.** Procédé selon l'une des revendications 1 à 4 caractérisé par le fait que le composé phénolique répond à la formule générale (I) laquelle R' représente un atome d'hydrogène et l'un des radicaux $R_1$, $R_2$, $R_3$ et $R_4$ représente un groupe hydroxyle, un radical méthyle ou un radical méthoxy et les 3 autres représentent un atome d'hydrogène.

**6.** Procédé selon l'une des revendications 1 à 5 caractérisé par le fait que le composé phénolique est le phénol, l'anisole, l'orthocrésol, le métacrésol, le 2-méthoxyphénol, le 2-éthylphénol, le 3-éthylphénol, 2-propylphénol, le 2-sec-butylphénol, le 2-tert-butylphénol, le 3-tert-butylphénol, le 2-méthoxyphénol, le 3-méthoxyphénol, le salicylate de méthyle, le 2-chlorophénol, le 3-chloro-phénol ; le 2,3-diméthylphénol, le 2,5-diméthylphénol, le 2,6-diméthylphénol, le 3,5-diméthyphénol, le 2,3-dichlorophénol, le 2,5-dichlorophénol, le 2,6-dichlorophénol, le 3,5-dichlorophénol, le 2,6-ditert-butylphénol, le 3,5-ditert-butylphénol ; le 2,3,5-triméthylphénol, le 2,3,6-triméthylphénol, le 2,3,5-trichlorophénol, le 2,3,6-trichlorophénol ; le 1-hydroxynaphtalène ; le 2-phénoxyphénol, le 3-phénoxyphénol.

**7.** Procédé selon l'une des revendications 1 à 6 caractérisé par le fait que le composé cétonique non énolisable est un composé cétonique répondant à la formule générale (II) :

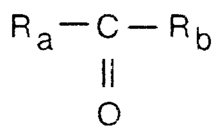

(II)

dans ladite formule (II) :

- $R_a$ et $R_b$, identiques ou différents sont des radicaux hydrocarbonés ayant chacun de 3 à 30 atomes de carbone ; les atomes de carbone de chaque radical $R_a$ et $R_b$ en position $\alpha$ par rapport au groupe carbonyle étant des carbones tertiaires.

**8.** Procédé selon l'une des revendications 1 à 7 caractérisé par le fait que le composé cétonique répond à la formule générale (IIa) :

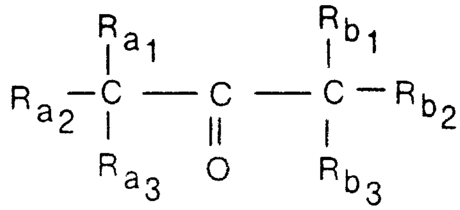

(IIa)

dans ladite formule (IIa) :

- $R_{a1}$, $R_{a2}$, $R_{a3}$ et $R_{b1}$, $R_{b2}$, $R_{b3}$ identiques ou différents, représentent des radicaux alkyle linéaires ou ramifiés ayant de 1 à 10 atomes de carbone, des radicaux cyclohexyle, phényle ou naphtyle éventuellement substitués,
- $R_{a1}$, $R_{a2}$, $R_{a3}$ et/ou $R_{b1}$, $R_{b2}$, $R_{b3}$ peuvent former ensemble et avec l'atome de carbone qui les porte un cycle benzénique ou naphtalénique éventuellement substitué.

**9.** Procédé selon l'une des revendications 1 à 8 caractérisé par le fait que le composé cétonique répond à la formule générale (IIb) :

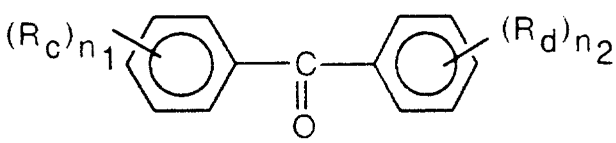

(IIb)

dans ladite formule (IIb) :

- $R_c$ et $R_d$ identiques ou différents représentent un atome d'hydrogène ou un substituant,
- $n_1$, $n_2$ identiques ou différents est un nombre égal à 0, 1, 2 ou 3.

**10.** Procédé selon l'une des revendications 1 à 9 caractérisé par le fait que le composé cétonique répond à la formule générale (IIb) dans laquelle $R_c$ et $R_d$, identiques ou différents représentent :

- des radicaux alkyle linéaires ou ramifies ayant de 1 à 4 atomes de carbone,
- un radical phényle,
- des radicaux alkoxy $R_{10}$ - O dans lesquels $R_{10}$ représente un radical alkyle linéaire ou ramifié ayant de 1 à 4 atomes de carbone ou le radical phényle,
- un groupe hydroxyle,
- un atome de fluor.

11. Procédé selon l'une des revendications 1 à 10 caractérisé par le fait que le composé cétonique répond à la formule générale (IIb) dans laquelle $R_c$ et $R_d$, identiques ou différents représentent un atome d'hydrogène ou un substituant tel que défini dans la revendication 10, de préférence en position 4,4' et $n_1$, $n_2$ identiques ou différents sont égaux à 0 ou 1.

12. Procédé selon l'une des revendications 1 à 11 caractérisé par le fait que le composé cétonique répond à la formule générale (IIb) dans laquelle $R_c$ et $R_d$, identiques ou différents représentent un atome d'hydrogène ; un radical méthyle, éthyle, tert-butyle, phényle ; un radical méthoxy ou éthoxy ; un groupe hydroxyle, de préférence en position 3,3' ou 4,4'.

13. Procédé selon l'une des revendications 1 à 12 caractérisé par le fait que le composé cétonique de formule générale (IIb) est :

- la benzophénone
- la méthyl-2 benzophénone
- la diméthyl-2,4 benzophénone
- la diméthyl-4,4' benzophénone
- la diméthyl-2,2' benzophénone
- la diméthoxy-4,4' benzophénone
- l'hydroxy-4 benzophénone
- la dihydroxy-4-4' benzophénone
- le benzoyl-4 biphényle

14. Procédé selon l'une des revendications 1 à 13 caractérisé par le fait que le catalyseur acide présente une fonction d'acidité - Ho comprise entre 5 et 20, de préférence entre 10 et 15.

15. Procédé selon l'une des revendications 1 à 13 caractérisé par le fait que le catalyseur acide est un acide protonique fort présentant un pKa dans l'eau inférieur à - 0,1 et, de préférence, inférieur à - 1,0, de préférence l'acide fluorhydrique, l'acide perchlorique, l'acide trifluorométhanesulfonique, l'acide paratoluènesulfonique, l'acide chlorosulfonique, l'acide fluorosulfonique, l'acide méthanesulfonique, l'acide benzène sulfonique et encore plus préférentiellement, l'acide fluorhydrique, l'acide perchlorique, l'acide trifluorométhanesulfonique ou l'acide méthanesulfonique.

16. Procédé selon l'une des revendications 1 à 13 caractérisé par le fait que le catalyseur acide est une résine sulfonique, de préférence, un copolymère styrène-divinylbenzène sulfoné, un copolymère phénol-formaldéhyde sulfoné, un copolymère tétrafluoroéthylène-perfluoro[2-(fluorosulfonyléthoxy)-propyl] vinyl éther et, de préférence, les résines suivantes : TEMEX 50, AMBERLYST 15, AMBERLYST 35, AMBERLYST 36, DOWEX 50 W ; DUOLITE ARC 9359 : NAFION.

17. Procédé selon l'une des revendications 1 à 13 caractérisé par le fait que le catalyseur acide est choisi parmi : la silice-alumine, la silice-$Ga_2O_3$, la silice-$B_2O_3$ ; les argiles acides ; les argiles pontées ; les zéolithes naturelles ou synthétiques ; les phosphates de bore, d'aluminium ou de gallium ; les phosphates lamellaires de métaux tétravalents répondant à la formule $\alpha$-$M(HPO_4)_2$, $pH_2O$ dans laquelle M représente un métal tétravalent choisi parmi le titane, le germanium, le zirconium ou l'étain et p est un nombre inférieur à 2 ; les phosphonates lamellaires de métaux tétravalents, de préférence les phosphonates lamellaires de zirconium ; les oxydes rendus acides par un traitement,de préférence les oxydes sulfatés de titane, de zirconium ou de fer.

18. Procédé selon l'une des revendications 1 à 13 caractérisé par le fait que le catalyseur acide utilisé est choisi parmi :

- les argiles naturelles présentant une structure dite "TOT" ou tétraèdre-octaèdre-tétraèdre, de préférence de la classe des smectites, et encore plus préférentiellement les montmorillonites.
- les argiles commerciales, déjà acides ou non, ou traitée par une solution aqueuse d'un acide.
- les argiles pontées préparées à partir de smectites, de préférence à partir de beidellites, en particulier de beidellites synthètiques ou de montmorillonites ; le pontage de l'argile étant de préférence un pontage à l'aluminium.

19. Procédé selon l'une des revendications 1 à 13 caractérisé par le fait que le catalyseur acide est choisi parmi :

- les zéolithes naturelles telles que la chabazite, la clinoptilolite, l'érionite, la mordénite, la phillipsite, l'offrétite,
- les zéolithes synthétiques telles que la zéolithe ZSM-5, la zéolithe Y, la ferrierite la zéolithe X de type faujasite, a zéolithe de type L, la mordénite, la zéolithe ZSM-11 ; la mazzite, l'offrétite, de préférence une zéolithe US-Y ou une zéolithe ZSM-5.

**20.** Procédé selon la revendication 19 caractérisé par le fait que la zéolithe mise en oeuvre est une zéolithe acidifiée.

**21.** Procédé selon l'une des revendications 1 à 20 caractérisé par le fait que le composé soufré ionisable est un composé minéral soufré ou un composé organique soufré, de préférence choisi parmi :

- les halogénures de soufre,
- les thiosulfates d'ammonium, de métal alcalin ou alcalino-terreux,
- l'hydrogène sulfuré ou ses précurseurs,
- les mercaptans de préférence les alkylmercarptans, phénylmercaptans et phénylalkylmercaptans,
- les thioalcools,
- les thiophénols,
- les acides thioorganiques, leurs sels ou leurs esters,
- les résines échangeuses de cations modifiées par réaction avec une alkyl mercaptoamine, de préférence les résines à squelette polystyrénique porteuses de groupes sulfoniques ayant réagi avec une alkyl mercaptoamine, de préférence celle avec un groupe amine primaire et encore plus préférentiellement celle ayant de 1 à 4 atomes de carbone.

**22.** Procédé selon la revendication 21 caractérisé par le fait que le composé soufré est choisi parmi :

- le monochlorure de soufre ou le bichlorure de soufre,
- le thiosulfate d'ammonium, de sodium ou de potassium,
- l'hydrogène sulfuré ou le sulfure de calcium ou de potassium,
- l'éthylmercaptan, le butylmercaptan, le 1-nonylmercaptan, le benzylmercaptan,
- le 2-mercaptoéthanol, le 3-mercapto-2-butanol,
- le thiophénol, l'o-thiocrésol, le m-thiocrésol, le p-thiocrésol, le thioxylénol, le p-méthylthiophénol, l'o-éthylthiophénol, le thiohydroquinone, le thionaphtol,
- l'acide thioacétique, l'acide thiopropionique, l'acide thiolactique, l'acide thiosalicylique, l'acide 2-mercaptoéthanesulfonique, l'acide 3-mercaptopropanesulfonique, l'acide mercaptosuccinique, l'acide thioglycolique, le thioglycolate de méthyle, d'éthyle,
- les résines à squelette polystyrénique porteuses de groupes sulfoniques ayant réagi avec la 2-mercaptoéthylamine, la 2-mercaptoisopropylamine, la 3-mercaptobutylamine.

**23.** Procédé selon l'une des revendications 1 à 22 caractérisé par le fait que le rapport molaire entre le composé phénolique de formule (I) et le composé cétonique de formule (II) est compris entre 1 et 20, de préférence entre 1 et 10.

**24.** Procédé selon l'une des revendications 14 à 16 caractérisé par le fait que la quantité de catalyseur acide exprimée par le rapport d'équivalents de protons au nombre de moles de composé cétonique de formule (II) varie entre $1.10^{-3}$ et 1,0, de préférence entre $5.10^{-3}$ et 0,5.

**25.** Procédé selon l'une des revendications 14 et 15 caractérisé par le fait que ledit rapport est compris entre 1,0 et 20, de préférence entre 5,0 et 10 lorsque l'acide est utilisé comme milieu réactionnel.

**26.** Procédé selon l'une des revendications 17 à 20 caractérisé par le fait que la quantité de catalyseur acide mis en oeuvre représente en poids par rapport au composé phénolique de formule (I) engagé, de 0,1 à 20 %, de préférence de 0,5 à 10 %.

**27.** Procédé selon l'une des revendications 1 à 26 caractérisé par le fait que la quantité de composé soufré mis en oeuvre exprimée par rapport au composé cétonique de formule (II) est telle que le rapport molaire entre le composé soufré et le composé cétonique de formule (II) soit compris entre 0,001 et 1,0, de préférence entre 0,005 et 0,20.

**28.** Procédé selon l'une des revendications 1 à 27 caractérisé par le fait que la température de la réaction est comprise entre 45°C et 200°C, de préférence entre 60°C et 150°C.

**29.** Procédé selon l'une des revendications 1 à 28 caractérisé par le fait que l'on prépare la diphénylfuchsone par réaction du phénol et de la benzophénone ; l'acide rosolique par réaction du phénol et de la dihydroxy-4,4' benzophénone ; la diphénylméthylfuchsone par réaction de l'ortho-crésol et de la benzophénone.

## Claims

**1.** A process for the preparation of a p-fuchsone, characterised in that it comprises reacting a phenolic compound having at least one hydrogen atom in the para position and a non-enolisable ketonic compound in the presence of an effective amount of a protonic acid having an acidity-Ho function of at least 5 and possibly in the presence of an effective amount of an ionisable sulphurous compound.

**2.** A process according to claim 1, characterised in that the phenolic compound having at least one hydrogen atom in the para position is a phenolic compound corresponding to the following general formula (I):

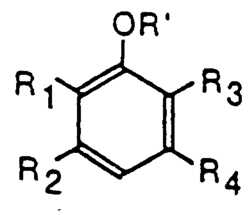
(I)

in which formula (I):

- the para position is free,
- $R_1$, $R_2$, $R_3$ and $R_4$ which are identical or different represent a hydrogen atom or any substituent,
- two groups $R_1$ and $R_2$ and/or $R_3$ and $R_4$ placed on two neighbouring carbon atoms can form together and with the carbon atoms which bear them a ring,
- R' represents a hydrogen atom or a hydrocarbon radical having 1 to 24 carbon atoms, which may be a branched or straight chain, saturated or unsaturated acyclic aliphatic radical; a monocyclic or polycyclic, saturated or unsaturated cycloaliphatic radical; a branched or straight chain, saturated or unsaturated aliphatic radical bearing a cyclic substituent.

**3.** A process according to one of claims 1 and 2, characterised in that the phenolic compound corresponds to general formula (I) in which:

- the radical R' represents one of the following groups:
  - a hydrogen atom
  - a branched or straight chain alkyl radical having from 1 to 6 carbon atoms, preferably from 1 to 4 carbon atoms
  - a cyclohexyl radical
  - a phenyl radical
  - a benzyl radical
- $R_1$, $R_2$, $R_3$ and $R_4$ which are identical or different represent $R_0$, one of the following groups:
  - a hydrogen atom,
  - a branched or straight chain alkyl radical having from 1 to 6 carbon atoms, preferably from 1 to 4 carbon atoms, such as methyl, ethyl, propyl, isopropyl, butyl, sec-butyl, tert-butyl,
  - a branched or straight chain alkenyl radical having from 2 to 6 carbon atoms, preferably from 2 to 4 carbon atoms, such as vinyl, allyl,
  - a branched or straight chain alkoxy radical having from 1 to 6 carbon atoms, preferably from 1 to 4 carbon atoms, such as the methoxy, ethoxy, propoxy, isopropoxy, butoxy radicals,
  - an acyl group having from 2 to 6 carbon atoms,
  - a radical of the formula:

$-R_5-OH$

$-R_5-COOR_6$

$-R_5-X$

$-R_5-CF_3$

in which formulae, $R_5$ represents a valence bond or a saturated or unsaturated, branched or straight chain, divalent hydrocarbon radical having from 1 to 6 carbon atoms, such as methylene, ethylene, propylene, isopropylene, isopropilydene, for example; $R_6$ represents a branched or straight chain alkyl radical having 1 to 6 carbon atoms; X symbolises a halogen atom, preferably a chlorine, bromine or fluorine atom.

- $R_1$, $R_2$, $R_3$ and $R_4$ which are identical or different represent $R_7$, one of the following more complex radicals:
  - a saturated or unsaturated carbocyclic radical having from 4 to 7 carbon atoms, preferably a cyclohexyl radical,
  - a radical of the formula

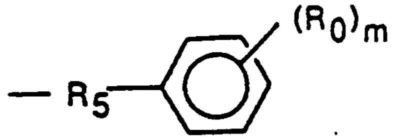

in which $R_5$ represents a valence bond or a saturated or unsaturated, branched or straight chain, divalent hydrocarbon radical having from 1 to 6 carbon atoms, such as methylene, ethylene, propylene, isopropylene, isopropylidene, for example, and $R_0$ having the meaning given hereinabove, and m is an integer of from 0 to 4,

  - a radical - $R_5$ - A - $R_8$, in which $R_5$ has the meaning given hereinabove, $R_8$ represents a branched or straight chain alkyl radical having from 1 to 6 carbon atoms or a radical of the formula

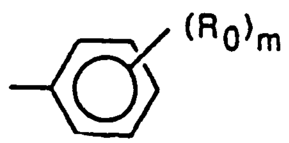

and A symbolises one of the following groups: -O-, -COO-, -OCOO-, $-SO_2-$,

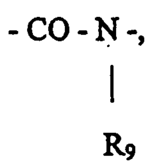

in which formulae, $R_9$ represents a hydrogen atom or an alkyl radical having from 1 to 4 carbon atoms, a cyclohexyl or phenyl radical,

- two groups $R_1$ and $R_2$ and/or $R_3$ and $R_4$ placed on two neighbouring carbon atoms can form together and with the carbon atom which bear them an unsaturated or aromatic carbocycle having from 4 to 7 carbon atom, and preferably 6 carbon atoms.

**4.** A process according to one of claims 1 to 3, characterised in that the phenolic compound corresponds to general formula (I) in which:

- R' represents a hydrogen atom
- $R_1$, $R_2$, $R_3$ and $R_4$ which are identical or different represent one of the following groups:

  - a hydrogen atom,
  - a branched or straight chain alkyl radical having from 1 to 4 carbon atoms,
  - a branched or straight chain alkoxy radical having from 1 to 4 carbon atoms,
  - a hydroxyl group,
  - a halogen atom,
  - a group $-CF_3$,
  - a cyclohexyl radical,
  - a phenyl radical,

- two groups $R_1$ and $R_2$ and/or $R_3$ and $R_4$ placed on two neighbouring carbon atoms can form together and with the carbon atoms which bear them a benzene ring.

**5.** A process according to one of Claims 1 to 4, characterised in that the phenolic compound corresponds to the general formula (I) in which R' represents a hydrogen atom, and one of the radicals $R_1$, $R_2$, $R_3$ and $R_4$ represents a hydroxyl group, a methyl radical or a methoxy radical, and the 3 others represent a hydrogen atom.

**6.** A process according to one of Claims 1 to 5, characterised in that the phenolic compound is phenol, anisol, orthocresol, metacresol, 2-methoxyphenol, 2-ethylphenol, 3-ethylphenol, 2-propylphenol, 2-sec-butylphenol, 2-tert-butylphenol, 3-tert-butylphenol, 2-methoxyphenol, 3-methoxyphenol, methyl salicylate, 2-chlorophenol, 3-chlorophenol; 2,3-dimethylphenol, 2,5-dimethylphenol, 2,6-dimethylphenol, 3,5-dimethylphenol, 2,3-dichlorophenol, 2,5-dichlorophenol, 2,6-dichlorophenol, 3,5-dichlorophenol, 2,6-ditert-butylphenol, 3,5-ditert-butylphenol; 2,3,5-trimethylphenol, 2,3,6-trimethylphenol, 2,3,5-trichlorophenol, 2,3,6-trichlorophenol; 1-hydroxynaphthalene; 2-phenoxyphenol, 3-phenoxyphenol.

**7.** A process according to one of Claims 1 to 6, characterised in that the non-enolisable ketonic compound is a ketonic compound corresponding to general formula (II):

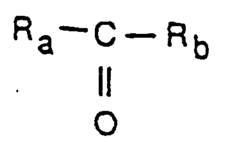

(II)

in which formula (II):

- $R_a$ and $R_b$ which are identical or different are hydrocarbon radicals each having from 3 to 30 carbon atoms; the carbon atoms of each radical $R_a$ and $R_b$ in position $\alpha$ with respect to the carbonyl group being tertiary carbons.

**8.** A process according to one of Claims 1 to 7, characterised in that the ketonic compound corresponds to general formula (IIa):

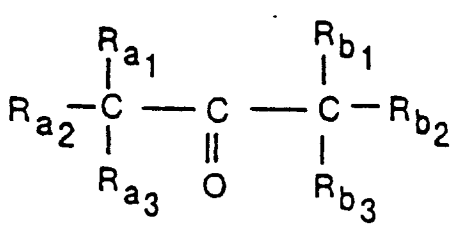

(IIa)

in which formula (IIa):

- $R_{a1}$, $R_{a2}$, $R_{a3}$ and $R_{b1}$, $R_{b2}$, $R_{b3}$ which are identical or different represent branched or straight chain alkyl radicals having from 1 to 10 carbon atoms, optionally substituted cyclohexyl, phenyl or naphthyl radicals,
- $R_{a1}$, $R_{a2}$, $R_{a3}$ and/or $R_{b1}$, $R_{b2}$, $R_{b3}$ can form together and with the carbon atom which bears them an optionally substituted naphthalene or benzene ring.

**9.** A process according to Claims 1 to 8, characterised in that the ketonic compound corresponds to general formula (IIb):

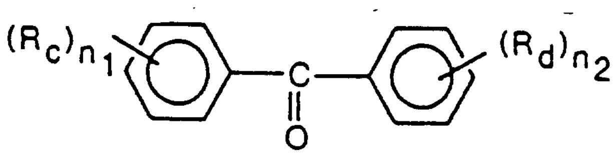

(IIb)

in which formula (IIb):

- $R_c$ and $R_d$ which are identical or different represent a hydrogen atom or a substituent,
- $n_1$, $n_2$ which are identical or different is a number equal to 0, 1, 2 or 3.

**10.** A process according to one of Claims 1 to 9, characterised in that the ketonic compound corresponds to general formula (IIb) in which $R_c$ and $R_d$ which are identical or different represent:

- branched or straight chain alkyl radicals having from 1 to 4 carbon atoms,
- a phenyl radical,
- alkoxy radicals $R_{10}$ - O in which $R_{10}$ represents a branched or straight chain alkyl radical having from 1 to 4 carbon atoms or the phenyl radical,
- a hydroxyl group,
- a fluorine atom.

**11.** A process according to one of Claims 1 to 10, characterised in that the ketonic compound corresponds to general formula (IIb) in which $R_c$ and $R_{d'}$ which are identical or different represent a hydrogen atom or a substituent such as defined in Claim 10, preferably in position 4,4', and $n_1$, $n_2$ which are identical or different are equal to 0 or 1.

**12.** A process according to one of Claims 1 to 11, characterised in that the ketonic compound corresponds to general formula (IIb) in which $R_c$ and $R_{d'}$ which are identical or different represent a hydrogen atom; a methyl, ethyl, tert-butyl or phenyl radical; a methoxy or ethoxy radical; a hydroxyl group, preferably in position 3,3' or 4,4'.

**13.** A process according to one of Claims 1 to 12, characterised in that the ketonic compound of general formula (IIb) is:

- benzophenone
- 2-methyl benzophenone
- 2,4-dimethyl benzophenone
- 4,4'-dimethyl benzophenone
- 2,2'-dimethyl benzophenone
- 4,4'-dimethoxy benzophenone
- 4-hydroxy benzophenone
- 4,4'-dihydroxy benzophenone
- 4-benzoyl biphenyl

**14.** A process according to one of Claims 1 to 13, characterised in that the acid catalyst has an acidity-Ho function of between 5 and 20, preferably of between 10 and 15.

**15.** A process according to one of Claims 1 to 13, characterised in that the acid catalyst is a strong protonic acid having a pKa in water of less than - 0.1, and preferably of less than - 1.0, preferably hydrofluoric acid, perchloric acid, trifluoromethanesulphonic acid, paratoluenesulphonic acid, chlorosulphonic acid, fluorosulphonic acid, methanesulphonic acid, benzenesulphonic acid, and, still more preferably, hydrofluoric acid, perchloric acid, trifluoromethanesulphonic acid, or methanesulphonic acid.

**16.** A process according to one of Claims 1 to 13, characterised in that the acid catalyst is a sulphonic resin, preferably a sulphonated styrene-divinylbenzene copolymer, a sulphonated phenol-formaldehyde copolymer, a tetrafluoroethylene-perfluoro[2-(fluorosulphonylethoxy)-propyl] vinyl ether copolymer, and, preferably, the following resins: TEMEX 50, AMBERLYST 15, AMBERLYST 35, AMBERLYST 36, DOWEX 50 W; DUOLITE ARC 9359: NAFION.

**17.** A process according to one of Claims 1 to 13, characterised in that the acid catalyst is selected from: silica-alumina, silica-$Ga_2O_3$, silica-$B_2O_3$; acid clays; bridged clays; natural or synthetic zeolites; boron, aluminium or gallium phosphates; lamellar phosphates of tetravalent metals corresponding to the formula $\alpha$-$M(HPO_4)_2$, $pH_2O$ in which M represents a tetravalent metal selected from titanium, germanium, zirconium or tin and p is a number less than 2: lamellar phosphonates of tetravalent metals, preferably lamellar phosphonates of zirconium; oxides which are made acid by a treatment, preferably the sulphated oxides of titanium, zirconium or iron.

**18.** A process according to one of Claims 1 to 13, characterised in that the acid catalyst used is selected from:

- natural clays having a structure referred to as "TOT" or tetrahedral-octahedral-tetrahedral, preferably from the class of smectites, and, still more preferably, montmorillonites,
- commercial clays, already acid or not, or treated with an aqueous solution of an acid.
- bridged clays prepared from smectites, preferably from beidellites, in particular from synthetic beidellites or montmorillonites; bridging of the clay preferably being an aluminium bridging.

**19.** A process according to one of Claims 1 to 13, characterised in that the acid catalyst is selected from:

- natural zeolites such as chabazite, clinoptilolite, erionite, mordenite, phillipsite and offretite, and
- synthetic zeolites, such as zeolite ZSM-5, zeolite Y, ferrierite, zeolite X of faujasite type, zeolite of type L, mordenite, zeolite ZSM-11, mazzite, offretite, preferably a zeolite US-Y or a zeolite ZSM-5.

**20.** A process according to Claim 19, characterised in that the zeolite used is an acidified zeolite.

**21.** A process according to one of Claims 1 to 20, characterised in that the ionisable sulphurous compound is a sulphurous mineral compound or a sulphurous organic compound, preferably selected from:

- sulphur halides,
- thiosulphides of ammonium, alkali metal or alkaline earth metal,
- hydrogen sulphide or its precursors,
- mercaptans, preferably alkylmercaptans, phenylmercaptans and phenylalkylmercaptans,
- thioalcohols
- thiophenols
- thioorganic acids, their salts or their esters,
- cation exchange resins modified by reaction with an alkyl mercaptoamine, preferably resins with a polystyrene skeleton bearing sulphonic groups having reacted with an alkyl mercaptoamine, preferably that with a primary amine group, and, still more preferably, that having from 1 to 4 carbon atoms.

**22.** A process according to Claim 21, characterised in that the sulphurous compound is selected from:

- sulphur monochloride or sulphur bichloride,
- ammonium, sodium or potassium thiosulphate,
- hydrogen sulphide or calcium or potassium sulphide,
- ethylmercaptan, butylmercaptan, 1-nonylmercaptan, benzylmercaptan,
- 2-mercaptoethanol, 3-mercapto-2-butanol,
- thiophenol, o-thiocresol, m-thiocresol, p-thiocresol, thioxylenol, p-methylthiophenol, o-ethylthiophenol, thiohydroquinone, thionaphthol,
- thioacetic acid, thiopropionic acid, thiolactic acid, thiosalicylic acid, 2-mercaptoethanesulphonic acid, 3-mercaptopropanesulphonic acid, mercaptosuccinic acid, thioglycolic acid, methyl and ethyl thioglycolate,
- resins with a polystyrene skeleton bearing sulphonic groups which have reacted with 2-mercaptoethylamine, 2-mercaptoisopropylamine, 3-mercaptobutylamine.

**23.** A process according to one of Claims 1 to 22, characterised in that the molar ratio between the phenolic compound of formula (I) and the ketonic compound of formula (II) is between 1 and 20, preferably between 1 and 10.

**24.** A process according to one of Claims 14 to 16, characterised in that the amount of acid catalyst expressed by the ratio of equivalents of protons to the number of moles of ketonic compound of formula (II) varies between $1.10^{-3}$ and 1.0, preferably between $5.10^{-3}$ and 0.5.

**25.** A process according to one of Claims 14 and 15, characterised in that said ratio is between 1.0 and 20, preferably between 5.0 and 10 when the acid is used as the reaction medium.

**26.** A process according to one of Claims 17 to 20, characterised in that the amount of acid catalyst used represents by weight in relation to the phenolic compound of formula (I) used, from 0.1 to 20%, preferably from 0.5 to 10%.

**27.** A process according to one of Claims 1 to 26, characterised in that the amount of sulphurous compound used, expressed in relation to the ketonic compound of formula (II), is such that the molar ratio between the sulphurous compound and the ketonic compound of formula (II) is between 0.001 and 1.0, preferably between 0.005 and 0.20.

**28.** A process according to one of Claims 1 to 27, characterised in that the reaction temperature is between 45°C and 200°C, preferably between 60°C and 150°C.

**29.** A process according to one of Claims 1 to 28, characterised in that the diphenylfuchsone is prepared by reacting phenol and benzophenone; the rosolic acid by reacting phenol and 4,4'-dihydroxybenzophenone; the diphenylmethylfuchsone by reacting ortho-cresol and benzophenone.

**Patentansprüche**

**1.** Verfahren zur Herstellung eines para-Fuchsons, dadurch gekennzeichnet, daß man eine Phenolverbindung mit mindestens einem Wasserstoffatom in der para-Position und eine nicht enolisierbare Ketoverbindung in Gegenwart einer wirksamen Menge einer Protonensäure mit einer Aciditätsfunktion $-H_0$ von mindestens 5 und gegebenenfalls in Gegenwart einer wirksamen Menge einer ionisierbaren Schwefelverbindung miteinander reagieren läßt.

**2.** Verfahren nach Anspruch 1, dadurch gekennzeichnet, daß die Phenolverbindung, die mindestens ein Wasserstoffatom in der para-Position aufweist, eine Phenolverbindung der allgemeinen Formel (I)

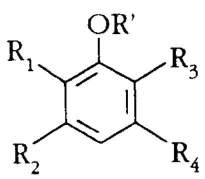

(I)

ist, wobei in der Formel (I):

- die para-Position frei ist,
- $R_1$, $R_2$, $R_3$ und $R_4$, identisch oder verschieden, ein Wasserstoffatom oder einen beliebigen Substituenten darstellen,
- zwei Gruppen $R_1$ und $R_2$ und/oder $R_3$ und $R_4$, die an zwei benachbarten Kohlenstoffatomen befindlich sind, gemeinsam und mit den Kohlenstoffatomen, die sie tragen, einen Ring bilden können,
- R' ein Wasserstoffatom oder einen Kohlenwasserstoffrest mit 1 bis 24 Kohlenstoffatomen darstellt, der ein gesättigter oder ungesättigter, linearer oder verzweigter, acyclischer aliphatischer Rest; ein gesättigter oder ungesättigter, monocyclischer oder polycyclischer cycloaliphatischer Rest; ein gesättigter oder ungesättigter, linearer oder verzweigter aliphatischer Rest sein kann, der einen ringförmigen Substituenten enthält.

**3.** Verfahren nach Anspruch 1 oder 2, dadurch gekennzeichnet, daß die Phenolverbindung der allgemeinen Formel (I) entspricht, bei der:

- der Rest R' eine der folgenden Gruppen darstellt:
  - ein Wasserstoffatom,
  - einen linearen oder verzweigten Alkylrest mit 1 bis 6 Kohlenstoffatomen, vorzugsweise mit 1 bis 4 Kohlenstoffatomen,

- einen Cyclohexylrest,
- einen Phenylrest,
- einen Benzylrest,

- $R_1$, $R_2$, $R_3$ und $R_4$, identisch oder verschieden, $R_0$ darstellen, hierunter eine der folgenden Gruppen:

  - ein Wasserstoffatom,
  - einen linearen oder verzweigten Alkylrest mit 1 bis 6 Kohlenstoffatomen, vorzugsweise mit 1 bis 4 Kohlenstoffatomen, wie z.B. Methyl, Ethyl, Propyl, Isopropyl, Butyl, sek.-Butyl oder tert.-Butyl,
  - einen linearen oder verzweigten Alkenylrest mit 2 bis 6 Kohlenstoffatomen, vorzugsweise mit 2 bis 4 Kohlentoffatomen, so z.B. Vinyl oder Allyl,
  - einen linearen oder verzweigten Alkoxyrest mit 1 bis 6 Kohlenstoffatomen, vorzugsweise mit 1 bis 4 Kohlenstoffatomen, so z.B. einen Methoxy-, Ethoxy-, Propoxy-, Isopropoxy- oder Butoxyrest,
  - eine Acylgruppe mit 2 bis 6 Kohlenstoffatomen,
  - einen Rest der Formel:

$$-R_5\text{-OH}$$

$$-R_5\text{-COOR}_6$$

$$-R_5\text{-X}$$

$$-R_5\text{-CF}_3$$

  wobei in diesen Formeln $R_5$ eine Valenzbindung oder einen gesättigten oder ungesättigten, linearen oder verzweigten, zweiwertigen Kohlenwasserstoffrest mit 1 bis 6 Kohlenstoffatomen darstellt, so z.B. Methylen, Ethylen, Propylen, Isopropylen, Isopropyliden; $R_6$ einen linearen oder verzweigten Alkylrest mit 1 bis 6 Kohlenstoffatomen darstellt; X ein Halogenatom darstellt, vorzugsweise ein Chlor-, Brom- oder Fluoratom,

- $R_1$, $R_2$, $R_3$ und $R_4$, identisch oder verschieden, $R_7$ darstellen, hierunter einen der folgenden komplexeren Reste:

  - einen gesättigten oder ungesättigten carbocyclischen Rest mit 4 bis 7 Kohlenstoffatomen, vorzugsweise einen Cyclohexylrest,
  - einen Rest der Formel

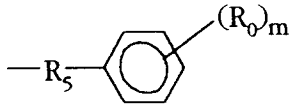

  - wobei in dieser Formel $R_5$ eine Valenzbindung oder einen zweiwertigen, linearen oder verzweigten, gesättigten oder ungesättigten Kohlenwasserstoffrest mit 1 bis 6 Kohlenstoffatomen darstellt, so z.B. Methylen, Ethylen, Propylen, Isopropylen oder Isopropyliden, und $R_0$ die zuvor angegebene Bedeutung hat und m eine ganze Zahl von 0 bis 4 ist,
  - einen Rest - $R_5$ - A - $R_8$, wobei in dieser Formel $R_5$ die zuvor angegebene Bedeutung hat, $R_8$ einen linearen oder verzweigten Alkylrest mit 1 bis 6 Kohlenstoffatomen oder einen Rest der folgenden Formel

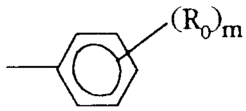

darstellt und A eine der folgenden Gruppen darstellt: -O-, -COO-, -OCOO-, $-SO_2-$,

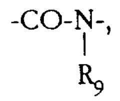

wobei in diesen Formeln $R_9$ ein Wasserstoffatom oder einen Alkylrest mit 1 bis 4 Kohlenstoffatomen, einen Cyclohexylrest oder einen Phenylrest darstellt,

- zwei Gruppen $R_1$ und $R_2$ und/oder $R_3$ und $R_4$, die an zwei benachbarten Kohlenstoffatomen befindlich sind, gemeinsam und mit den Kohlenstoffatomen, die sie tragen, einen aromatischen oder ungesättigten Carbocyclus mit 4 bis 7 Kohlenstoffatomen, vorzugsweise mit 6 Kohlenstoffatomen, bilden können.

**4.** Verfahren nach einem der Ansprüche 1 bis 3, dadurch gekennzeichnet, daß die Phenolverbindung der allgemeinen Formel (I) entspricht, in der:

- R' ein Wasserstoffatom darstellt,
- $R_1$, $R_2$, $R_3$ und $R_4$, identisch oder verschieden, eine der folgenden Gruppen darstellen:
    - ein Wasserstoffatom,
    - einen linearen oder verzweigten Alkylrest bis 1 bis 4 Kohlenstoffatomen,
    - einen linearen oder verzweigten Alkoxyrest mit 1 bis 4 Kohlenstoffatomen,
    - eine Hydroxylgruppe,
    - ein Halogenatom,
    - eine $CF_3$-Gruppe,
    - einen Cyclohexylrest,
    - einen Phenylrest,
- zwei Gruppen $R_1$ und $R_2$ und/oder $R_3$ und $R_4$, die an zwei benachbarten Kohlenstoffatomen befindlich sind, gemeinsam und mit den Kohlenstoffatomen, die sie tragen, einen Benzolring bilden können.

**5.** Verfahren nach einem der Ansprüche 1 bis 4, dadurch gekennzeichnet, daß die Phenolverbindung der allgemeinen Formel (I) entspricht, wobei in dieser Formel R' ein Wasserstoffatom darstellt und einer der Reste $R_1$, $R_2$, $R_3$ und $R_4$ eine Hydroxylgruppe, einen Methylrest oder einen Methoxyrest darstellt und die 3 anderen ein Wasserstoffatom darstellen.

**6.** Verfahren nach einem der Ansprüche 1 bis 5, dadurch gekennzeichnet, daß die Phenolverbindung Phenol, Anisol, ortho-Kresol, meta-Kresol, 2-Methoxyphenol, 2-Ethylphenol, 3-Ethylphenol, 2-Propylphenol, 2-sek.-Butylphenol, 2-tert.-Butylphenol, 3-tert.-Butylphenol, 2-Methoxyphenol, 3-Methoxyphenol, Methylsalicylat, 2-Chlorphenol, 3-Chlorphenol, 2,3-Dimethylphenol, 2,5-Dimethylphenol, 2,6-Dimethylphenol, 3,5-Dimethylphenol, 2,3-Dichlorphenol, 2,5-Dichlorphenol, 2,6-Dichlorphenol, 3,5-Dichlorphenol, 2,6-Di-tert.-butylphenol, 3,5-Di-tert.-butylphenol; 2,3,5-Trimethylphenol, 2,3,6-Trimethylphenol, 2,3,5-Trichlorphenol, 2,3,6-Trichlorphenol; 1-Hydroxynaphthalin; 2-Phenoxyphenol oder 3-Phenoxyphenol ist.

**7.** Verfahren nach einem der Ansprüche 1 bis 6, dadurch gekennzeichnet, daß die nicht enolisierbare Ketoverbindung eine Ketoverbindung der allgemeinen Formel (II)

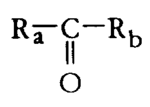

(II)

ist, wobei in der Formel (II):

- $R_a$ und $R_b$, identisch oder verschieden, Kohlenwasserstoffreste mit jeweils 3 bis 30 Kohlenstoffatomen sind, wobei die Kohlenstoffatome jedes Restes $R_a$ und $R_b$ in $\alpha$-Position zur Carbonylgruppe tertiäre Kohlenstoffe

sind.

8. Verfahren nach einem der Ansprüche 1 bis 7, dadurch gekennzeichnet, daß die Ketoverbindung der allgemeinen Formel (IIa) entspricht

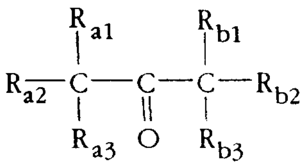

(IIa)

wobei in der Formel (IIa):

- $R_{a1}$, $R_{a2}$, $R_{a3}$ und $R_{b1}$, $R_{b2}$, $R_{b3}$, identisch oder verschieden, lineare oder verzweigte Alkylreste mit 1 bis 10 Kohlenstoffatomen, Cyclohexyl-, Phenyl- oder Naphthylreste darstellen, die gegebenenfalls substituiert sind;
- $R_{a1}$, $R_{a2}$, $R_{a3}$ und/oder $R_{b1}$, $R_{b2}$, $R_{b3}$ gemeinsam und mit den Kohlenstoffatomen, das sie trägt, einen Benzolring oder einen Naphthalinring bilden können, der gegebenenfalls substituiert ist.

9. Verfahren nach einem der Ansprüche 1 bis 8, dadurch gekennzeichnet, daß die Ketoverbindung der allgemeinen Formel (IIb)

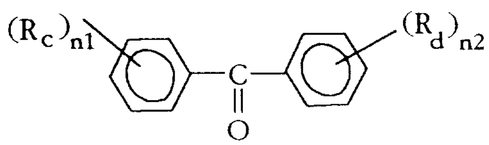

(IIb)

entspricht, wobei in der Formel (IIb):

- $R_c$ und $R_d$, identisch oder verschieden, ein Wasserstoffatom oder einen Substituenten darstellen;
- n1 und n2, identisch oder verschieden, eine Zahl 0, 1, 2 oder 3 sind.

10. Verfahren nach einem der Ansprüche 1 bis 9, dadurch gekennzeichnet, daß die Ketoverbindung der allgemeinen Formel (IIb) entspricht, in der $R_c$ und $R_d$, identisch oder verschieden, darstellen:

- einen linearen oder verzweigten Alkylrest mit 1 bis 4 Kohlenstoffatomen,
- einen Phenylrest,
- einen Alkoxyrest $R_{10}$ - O, wobei in dieser Formel $R_{10}$ einen linearen oder verzweigten Alkylrest mit 1 bis 4 Kohlenstoffatomen oder einen Phenylrest darstellt,
- eine Hydroxylgruppe,
- ein Fluoratom.

11. Verfahren nach einem der Ansprüche 1 bis 10, dadurch gekennzeichnet, daß die Ketoverbindung der allgemeinen Formel (IIb) entspricht, in der $R_c$ und $R_d$, identisch oder verschieden, ein Wasserstoffatom oder einen wie in Anspruch 10 definierten Substituenten darstellen, vorzugsweise in 4,4'-Position, und n1 und n2, identisch oder verschieden, 0 oder 1 sind.

12. Verfahren nach einem der Ansprüche 1 bis 11, dadurch gekennzeichnet, daß die Ketoverbindung der allgemeinen Formel (IIb) entspricht, wobei in der Formel $R_c$ und $R_d$, identisch oder verschieden, ein Wasserstoffatom; einen Methyl-, Ethyl-, tert.-Butyl-, Phenylrest; einen Methoxy- oder Ethoxyrest; eine Hydroxylgruppe, vorzugsweise in 3,3'- oder 4,4'-Position, darstellen.

13. Verfahren nach einem der Ansprüche 1 bis 12, dadurch gekennzeichnet, daß die Ketoverbindung der allgemeinen Formel (IIb) eine der folgenden Verbindungen ist:

- Benzophenon,
- 2-Methylbenzophenon
- 2,4-Dimethylbenzophenon
- 4,4'-Dimethylbenzophenon
- 2,2'-Dimethylbenzophenon
- 4,4'-Dimethoxybenzophenon
- 4-Hydroxybenzophenon
- 4,4'-Dihydroxybenzophenon
- 4-Benzoylbiphenyl.

**14.** Verfahren nach einem der Ansprüche 1 bis 13, dadurch gekennzeichnet, daß der saure Katalysator eine Aciditätsfunktion $-H_o$ zwischen 5 und 20, vorzugsweise zwischen 10 und 15, aufweist.

**15.** Verfahren nach einem der Ansprüche 1 bis 13, dadurch gekennzeichnet, daß der saure Katalysator eine starke Protonsäure mit einem $pK_a$-Wert in Wasser von weniger als -0,1 ist, vorzugsweise von weniger als -1,0, insbesondere Fluorwasserstoffsäure, Perchlorsäure, Trifluormethansulfonsäure, para-Toluolsulfonsäure, Chlorsulfonsäure, Fluorsulfonsäure, Methansulfonsäure, Benzolsulfonsäure, vor allem Fluorwasserstoffsäure, Perchlorsäure, Trifluormethansulfonsäure oder Methansulfonsäure.

**16.** Verfahren nach einem der Ansprüche 1 bis 13, dadurch gekennzeichnet, daß der saure Katalysator ein Sulfonsäureharz ist, vorzugsweise ein sulfoniertes Styrol/Divinylbenzol-Copolymer, ein sulfoniertes Phenol/Formaldehyd-Copolymer, ein Tetrafluorethylen/Perfluor[2-(fluorsulfonylethoxy)-propyl]-vinyl-ether-Copolymer, vorzugsweise eines der folgenden Harze: TEMEX 50, AMBERLYST 15, AMBERLYST 35, AMBERLYST 36, DOWEX 50 W, DUOLITE ARC 9359 : NAFION.

**17.** Verfahren nach einem der Ansprüche 1 bis 13, dadurch gekennzeichnet, daß der saure Katalysator ausgewählt ist aus: Siliciumoxid-Aluminiumoxid, Siliciumoxid-$Ga_2O_3$, Siliciumoxid-$B_2O_3$; sauren Tonen; verbrückten Tonen; natürlichen oder synthetischen Zeolithen; Bor-, Aluminium- oder Galliumphosphaten; plättchen- oder lamellenartigen Phosphaten vierwertiger Metalle, die der Formel $\alpha\text{-}M(HPO_4)_2 \cdot pH_2O$ entsprechen, in der M ein vierwertiges Metall darstellt, das ausgewählt ist aus Titan, Germanium, Zirkonium oder Zinn, und p eine Zahl kleiner als 2 ist; lamellenhaften Phosphonaten vierwertiger Metalle, vorzugsweise lamellenförmige Zirkoniumphosphonaten; Oxiden, die durch eine Behandlung sauer gemacht worden sind, vorzugsweise sulfathaltige Titan-, Zirkonium- oder Eisenoxiden.

**18.** Verfahren nach einem der Ansprüche 1 bis 13, dadurch gekennzeichnet, daß der verwendete saure Katalysator ausgewählt ist aus:

- natürlichen Tonen mit einer sogenannten TOT-Struktur oder Tetraeder/Oktaeder/Tetraeder-Struktur, vorzugsweise aus der Klasse der Smektite, insbesondere der Montmorillonite;
- handelsüblichen Tonen, die bereits sauer sind oder auch nicht oder durch eine wäßrige Lösung einer Säure behandelt worden sind;
- verbrückten Tonen, hergestellt ausgehend von Smektiten, vorzugsweise ausgehend von Beidelliten, insbesondere von synthetischen Beidelliten oder Montmorilloniten, wobei die Verbrückung des Tons vorzugsweise eine Aluminiumverbrückung ist.

**19.** Verfahren nach einem der Ansprüche 1 bis 13, dadurch gekennzeichnet, daß der saure Katalysator ausgewählt ist aus:

- natürlichen Zeolithen wie Chabazit, Clinoptilolit, Erionit, Mordenit, Phillipsit und Offretit;
- synthetischen Zeolithen wie Zeolith ZSM-5, Zeolith Y, Ferrierit, Zeolith X vom Typ Faujasit, Zeolith vom Typ L, Mordenit, Zeolith ZSM-11; Mazzit, Offretit, vorzugsweise ein Zeolith US-Y oder ein Zeolith ZSM-5.

**20.** Verfahren nach Anspruch 19, dadurch gekennzeichnet, daß der eingesetzte Zeolith ein angesäuerter Zeolith ist.

**21.** Verfahren nach einem der Ansprüche 1 bis 20, dadurch gekennzeichnet, daß die ionisierbare schwefelhaltige Verbindung eine anorganische schwefelhaltige Verbindung oder eine organische schwefelhaltige Verbindung ist, die vorzugsweise ausgewählt ist aus:

- Schwefelhalogeniden,
- Ammonium-, Alkalimetall- oder Erdalkalimetallthiosulfaten,
- Schwefelwasserstoff oder seinen Vorläufern,
- Mercaptanen, vorzugsweise Alkylmercaptanen, Phenylmercaptanen und Phenylalkylmercaptanen,
- Thioalkoholen,
- Thiophenolen,
- thioorganischen Säuren, ihren Salzen oder ihren Estern,
- Kationenaustauscherharzen, die durch Reaktion mit einem Alkylmercaptoamin modifiziert sind, vorzugsweise Harzen mit einem Polystyrolgerüst, die Sulfonsäuregruppen enthalten, die mit einem Alkylmercaptoamin reagiert haben, vorzugsweise solchen mit einer primären Amingruppe und insbesondere solchen mit 1 bis 4 Kohlenstoffatomen.

**22.** Verfahren nach Anspruch 21, dadurch gekennzeichnet, daß die schwefelhaltige Verbindung ausgewählt ist aus:

- Schwefelmonochlorid oder Schwefeldichlorid,
- Ammonium-, Natrium- oder Kaliumthiosulfat,
- Schwefelwasserstoff oder Calcium- oder Kaliumsulfid,
- Ethylmercaptan, Butylmercaptan, 1-Nonylmercaptan, Benzylmercaptan,
- 2-Mercaptoethanol, 3-Mercapto-2-butanol,
- Thiophenol, ortho-Thiokresol, meta-Thiokresol, para-Thiokresol, Thioxylenol, para-Methylthiophenol, ortho-Ethylthiophenol, Thiohydrochinon, Thionaphthol,
- Thioessigsäure, Thiopropionsäure, Thiomilchsäure, Thiosalicylsäure, 2-Mercaptoethansulfonsäure, 3-Mercaptopropansulfonsäure, Mercaptobernsteinsäure, Thioglykolsäure, Methylthioglykolat und Ethylthioglykolat,
- Harzen mit einem Polystyrolgerüst, die Sulfonsäuregruppen enthalten, die mit 2-Mercaptoethylamin, 2-Mercaptoisopropylamin oder 3-Mercaptobutylamin reagiert haben.

**23.** Verfahren nach einem der Ansprüche 1 bis 22, dadurch gekennzeichnet, daß das Molverhältnis zwischen der Phenolverbindung der Formel (I) und der Ketoverbindung der Formel (II) zwischen 1 und 20 liegt, vorzugsweise zwischen 1 und 10.

**24.** Verfahren nach einem der Ansprüche 14 bis 16, dadurch gekennzeichnet, daß die Menge an saurem Katalysator, berechnet als das Verhältnis der Äquivalente der Protonen zur Molanzahl der Ketoverbindung der Formel (II), zwischen $1 \cdot 10^{-3}$ und 1,0 variiert, vorzugsweise zwischen $5 \cdot 10^{-3}$ und 0,5.

**25.** Verfahren nach Anspruch 14 oder 15, dadurch gekennzeichnet, daß das Verhältnis zwischen 1,0 und 20, vorzugsweise zwischen 5,0 und 10, liegt, wenn die Säure als Reaktionsmilieu verwendet wird.

**26.** Verfahren nach einem der Ansprüche 17 bis 20, dadurch gekennzeichnet, daß die Menge an eingesetztem saurem Katalysator, gewichtsbezogen in bezug auf die eingesetzte Phenolverbindung der Formel (I), 0,1 bis 20 % darstellt, vorzugsweise 0,5 bis 10 %.

**27.** Verfahren nach einem der Ansprüche 1 bis 26, dadurch gekennzeichnet, daß die Menge an eingesetzter schwefelhaltiger Verbindung, berechnet in bezug auf die Ketoverbindung der Formel (II), derart ist, daß das Molverhältnis zwischen schwefelhaltiger Verbindung und Ketoverbindung der Formel (II) zwischen 0,001 und 1,0 liegt, vorzugsweise zwischen 0,005 und 0,20.

**28.** Verfahren nach einem der Ansprüche 1 bis 27, dadurch gekennzeichnet, daß die Reaktionstemperatur zwischen 45 °C und 200 °C, vorzugsweise zwischen 60 °C und 150 °C, liegt.

**29.** Verfahren nach einem der Ansprüche 1 bis 28, dadurch gekennzeichnet, daß man das Diphenylfuchson durch Reaktion von Phenol und Benzophenon herstellt; man die Rosolsäure (Steinkohlenteersäure) durch Reaktion von Phenol und 4,4'-Dihydroxybenzophenon herstellt; man das Diphenylmethylfuchson durch Reaktion von ortho-Kresol und Benzophenon herstellt.

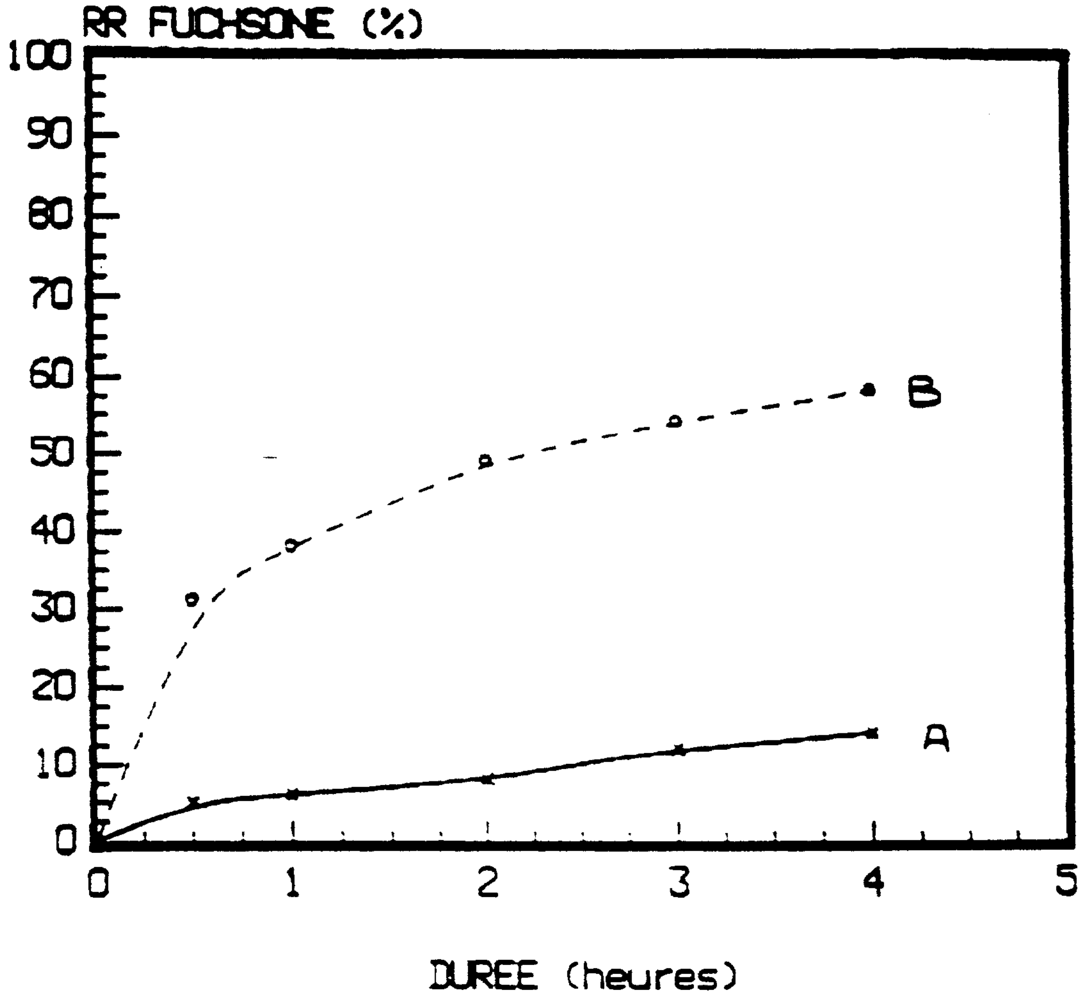
RR FUCHSONE (%)
100
90
80
70
60
50
40
30
20
10
0
0
1
2
3
4
5
B
A
DUREE (heures)